(19) **Europäisches Patentamt**
**European Patent Office**
**Office européen des brevets**

(11) **EP 4 521 411 A2**

(12) **EUROPEAN PATENT APPLICATION**

(43) Date of publication:
**12.03.2025 Bulletin 2025/11**

(21) Application number: **25152252.0**

(22) Date of filing: **19.09.2022**

(51) International Patent Classification (IPC):
**G16B 20/20** (2019.01)

(52) Cooperative Patent Classification (CPC):
(C-Sets available)
**C12Q 1/6869; C12Q 1/6855;** G16B 20/20;
G16B 30/10; G16B 40/30 (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.09.2021 IT 202100024101**

(62) Document number(s) of the earlier application(s) in
accordance with Art. 76 EPC:
**22782770.6 / 4 405 500**

(71) Applicant: **Menarini Silicon Biosystems S.p.A.
40013 Castel Maggiore (BO) (IT)**

(72) Inventors:
• **MANARESI, Nicolò
40013 Castel Maggiore (BO) (IT)**

• **FORCATO, Claudio
40013 Castel Maggiore (BO) (IT)**
• **FERRARINI, Alberto
40013 Castel Maggiore (BO) (IT)**

(74) Representative: **Studio Torta S.p.A.
Via Viotti, 9
10121 Torino (IT)**

Remarks:
This application was filed on 16.01.2025 as a
divisional application to the application mentioned
under INID code 62.

(54) **METHOD FOR ANALYSING THE DEGREE OF SIMILARITY OF AT LEAST TWO SAMPLES
USING DETERMINISTIC RESTRICTION-SITE WHOLE GENOME AMPLIFICATION (DRS-WGA)**

(57) The present disclosure relates to a method for analyzing the degree of similarity of at least two samples in a plurality of samples comprising genomic DNA. The method comprises the following steps. a) Providing a plurality of samples comprising genomic DNA. b) Carrying out, separately on each sample, a deterministic restriction-site whole genome amplification (DRS-WGA) of said genomic DNA, c) Preparing a massively parallel sequencing library using a fragmentation-free, sequencing-adaptor/WGA fusion-primer PCR reaction from each product of said DRS-WGA. d) Carrying out low-pass whole genome sequencing at a mean coverage depth of < 1x on said massively parallel sequencing library, e) Aligning for each sample the reads obtained in step d) on a reference genome, f) Extracting for each sample the allelic content at a plurality of polymorphic loci. g) Calculating a pair-wise similarity score for the at least two samples as a function of the allelic content measured at said plurality of loci. h) Determining the degree of similarity of the at least two samples on the basis of the similarity score.

FIG. 1

**(Cont. next page)**

(52) Cooperative Patent Classification (CPC): (Cont.)

C-Sets
**C12Q 1/6855, C12Q 2521/301, C12Q 2535/122;**
**C12Q 1/6869, C12Q 2521/301, C12Q 2525/191,**
**C12Q 2535/122**

## Description

Technical Field

The present disclosure relates to a method for sample pairing, assigning the identity of each of a plurality of samples to a class or individual, by analysing data obtained by low-pass whole genome sequencing carried out on said plurality of samples, achieving single-cell resolution, with or without the use of references.

In addition to sample pairing, the method provides a unified assay enabling the simultaneous identification and characterisation of a sample under-test among the samples.

The method according to the present disclosure can be used in several fields of application, including, but not limited to:

- single-cell forensic human identification
- sample-identification during the analysis of circulating tumor cells
- identification of fetal cells or fetal cell free DNA (cfDNA) in maternal body fluids for non-invasive prenatal testing
- identification of embryo cells or cfDNA in invasive preimplantation genetic testing (PGT)and non-invasive PGT on spent embryo medium
- identification of fetal component in prenatal diagnosis on invasively obtained samples and products of conception (e.g.: maternal or exogenous contamination assessment)
- molar pregnancy, multiple pregnancy (including Vanishing/Chimera), uniparental disomy (isodisomy or heterodisomy), ROH and consanguinity identification, non-disjunction error classification in material derived from the conceptus
- microchimerism
- cell line authentication (e.g.: stem cells).

Prior Art

## Sample identification and sample-pairing state of the art

The most wide-spread method for sample identification relies on the analysis of highly polymorphic Short Tandem Repeats (STR) loci (also called microsatellites). This method involves carrying out a targeted PCR for a plurality of loci and detecting amplicons with capillary electrophoresis. In human identification, since for each locus each allele (from maternal and paternal origin) can have many different values, a great diversity is generated with a relative low number of genetic loci amplified, such as that the allele sizes of an individual measured over 10 or 20 loci can identify with high probability an individual in a large cohort. Applying this method for single-cells can be challenging, especially if the quality of the DNA is low or degraded (for example, degraded due to fixation, or environmental conditions for storage, or other biological processes), as allelic drop-out can impair the retrieval of sufficient information to assign the sample identity. This holds true regardless of the fact that the multiplex PCR is carried out directly on a single-cell sample (thus consuming that sample) or on an aliquot of Whole Genome Amplification product from a single-cell, thus enabling repeated testing on different aliquots of the same WGA product.

Allelic drop-out can significantly decrease the alleles detected in the electropherogram of an STR assay down to 80%, 70%, 60%, 50%, 40%, 30%, 20%, 10% or lower. In addition, allelic drop-in can occur, resulting in additional peaks confounding the interpretation, especially for highly degraded samples and low-input template such as with single-cells. The resulting information is then insufficient to assign the sample identity with confidence.

The requirements for minimum number of alleles from STR loci depends on several factors but it is generally true, and known to those skilled in the art, that when matching a profile to a large population many more informative loci are required, while matching a sample to a smaller cohort of potential contributors represents a simpler problem which can be solved with lower number of detected alleles.

For example, in forensic caseworks, such as from sexual assault, DNA and cells from one or more perpetrators and the victim may be present, with a number of contributors which may be 1 victim and 1, 2, 3, 4, 5 or more perpetrators. In the case of multiple male perpetrators, the problem may be exacerbated by the fact that the target cells for analysis are sperm-cells which, being haploid, only have a single-allele per locus. When analyzing single-cells from a casework it may therefore become impossible to use single-cell information to reliably infer the number of contributors and assemble a reconstructed complete profile from that contributor under limited single-cell data.

As an example, single-sperm cells may be isolated using the DEPArray (Fontana et. al, "Isolation and genetic analysis of pure cells from forensic biological mixtures: The precision of a digital approach", Forensic Sciences International: Genetics 2007, http://dx.doi.org/10.1016/j.fsigen.2017.04.023), which allows to collect up to 48 single-sperms from a single DEPArray run, using the validated forensic application, or up to 96 single-cells using different application programs available from DEPArray system.

Single-cell forensic identification of different contributor profiles from a mixed evidence of blood in blood, using the DEPArray system to isolate individual cells has been demonstrated in K. Anslinger, B. Bayer, "Whose blood is it? Application of DEPArray™ technology for the identification of individual/s who contributed blood to a mixed stain" Int J Legal Med. 2019 Mar;133(2):419-426. doi: 10.1007/s00414-018-1912-7. Epub 2018 Aug 18.

In general the problem of reconstructing a complete profile and/or determining genetic information by in-silico reconstruction of a complete profile from a plurality of incomplete profiles of single cells, is harder to solve

(i) the lower the number of single-cells analyzed,
(ii) the lower the number of alleles detected per cell,
(iii) the higher the number of contributors,
(iv) the lower the level of representation of the minor contributor among analyzed cells.

Beyond single-cell forensic obtained by direct isolation of individual cells, other methods such as subsampling (K. Huffman, E. Hanson and J Ballantyne, "Recovery of single source DNA profiles from mixtures by direct single cell subsampling and simplified micromanipulation", Science & Justice Volume 61, Issue 1, January 2021, Pages 13-25) entail the analysis of a multiplicity of samples, composed of collections of small cell pools of e.g. 2 or 3 cells per pool. Also in this case it may be beneficial to have a system to identify if the pool is composed of cells from the same contributor or multiple contributors, and possibly identify the overall number of contributors among all pools, as well as enabling further genetic analysis on homogeneous pools, e.g. for additional investigative purposes such as determining ancestry or physical traits connected to genomic characteristics.

As a further example, cell lines authentication is commonly carried out using STR analysis. Most STR kits require capillary electrophoresis sequencers for fragment length analysis of the fluorescent amplification products. With the spread of massively parallel sequencers accessibility of capillary electrophoresis has declined, and many labs find themselves at a loss to analyze in-house STR profiles with capillary electrophoresis.

Targeted PCR panels for the analysis of STR using massively parallel sequencers are now available. However, this implies the acquisition of additional reagents often not already present in the lab.

As a further example, there is the need for sample identification and/or pairing in non-invasive prenatal diagnosis protocols based on isolation of fetal cells from maternal bodily fluid. These may be for example fetal cells (such as fetal nucleated red blood cells or trophoblasts) isolated from maternal blood. Given that cells are so rare there is a significant risk that the individual cells isolated from the enrichment process may be maternal cells as opposed to fetal cells due to several reasons, such as limited specificity in the immunofluorescence staining or ambiguous morphological selection, technical imperfections and errors in sorting equipment used in their isolation. Whatever the process and criteria used to isolate those cells, given the importance to ensure that the diagnosis is carried out on an actual fetal cell, it is essential to verify whether only fetal genetic material is the input of the genetic analysis, and to detect possible maternal contamination (admixed cells), or complete sample swapping (the single-cell is maternal), or even a contamination from e.g. the operator. While a mixed sample (e.g. 1 fetal cell 1 maternal cell, i.e. 50% contamination) may be still be acceptable for some chromosomal aneuploidy analysis, the lower purity may impair the detection of smaller aberrations like microdeletions, depending on the assay used.

Thus it is current practice in the state of the art to carry out STR analysis as an additional confirmatory test of fetal origin for the cell recovered during cell-based NIPD (Vossaert L, Wang Q, Salman R et al. "Validation Studies for Single Circulating Trophoblast Genetic Testing as a Form of Noninvasive Prenatal Diagnosis" American Journal of Human Genetics (2019) 105(6) 1262-1273; L.D. Jeppesen et al., "Cell-based non-invasive prenatal diagnosis in a pregnancy at risk of cystic fibrosis" Prenatal Diagnosis. 2020;1-7.; Manaresi et al., EP2152859B1).

In a recent paper (Zhuo X, Wang Q, Vossaert L, Salman R, Kim A, Van den Veyver I, et al. (2021) "Use of amplicon-based sequencing for testing fetal identity and monogenic traits with Single Circulating Trophoblast (SCT) as one form of cell-based NIPT" PLoS ONE 16(4): e0249695. https://doi.org/10.1371/journal.pone.0249695) it is recognized that "Whole genome shotgun (WGS) sequencing at low coverage (5-10 million reads per cell) provides good copy number data, but it does not readily distinguish fetal and maternal cells if the fetus is female". In this work, genotyping with a panel of 90 highly polymorphic SNPs using PCR-based target amplification (of 40 amplicons) and massively parallel sequencing is proposed as an alternative to STR analysis in order to confirm fetal origin of the cell recovered for diagnosis. This approach uses a small aliquot of DNA from the single-cell WGA product, however it still has the drawback of requiring additional samples work-up and associated costs, with respect to the workflow to assess aneuploidy based on low-pass WGS.

Non-invasive assessment of molar pregnancies and gestational trophoblastic disease has been demonstrated on circulating trophoblasts (Sunde L et al., "Hydatidiform mole diagnostics using circulating gestational trophoblasts isolated from maternal blood" Mol Genet Genomic Med. 2020;00:e1565. https: / /doi. org/10 .1002/mgg3 .1565), but STR analysis is once again considered essential to determine the origin of the rare trophoblasts isolated from maternal blood. Hydatidiform moles (HMs) can be "complete moles" which are typically diploid with both genome sets originating from the father (parental type: PP), due to a fertilization of an egg which has lost the maternal nucleus followed, in the majority of cases, by a duplication of the sperm chromosomes, or -in a minority of cases- by the fertilization by two sperms. Most of the HMs with the parental type PP show homozygosity in all loci (P1P1), whereas approximately 15% show heterozygosity in some loci (P1P2). Partial moles are HMs typically triploid with two genome sets from the father and one from the mother (parental type: PPM). Complete moles, carry an increased risk of Choriocarcinoma (15% with respect to 0.5% in partial moles). Thus, it is of interest to understand if the HMs carry a copy of the maternal genome or if it is absent.

As a further example of need for sample pairing methods, there is the identification for sample tracking in laboratory workflow. When sequencing multiple low-pass whole genome sequencing samples for genome-wide copy number profiling it may be beneficial to verify that there is no sample mix-up, and that patient sample code assignment in the Laboratory Management Information System (LIMS) is consistent with the patient assignment obtained from sequencing data.

Another example of need for sample pairing methods is the assessment of endothelial cell origin (host or donor) in patients of allogenic hematopoietic cell transplantation (allo-HSCT). Detecting donor-derived endothelial cells is of interest in the study of the physio-pathologic relationships between endothelium and graft-versus-host disease (GVHD), for the potential role of vascular endothelium as a target in early phase of GVHD and the potential tolerogenic role of donor-derived endothelial cells, as well as graft-versus-tumor (reviewed in Penack O. et al., "The importance of neovascularization and its inhibition for allogeneic hematopoietic stem cell transplantation" Blood, Volume 117, Issue 16, 21 April 2011, Pages 4181-4189). Sex-mismatched samples are often used in order to enable such analysis, but it would be desirable to have a method to analyze samples where host and donor have the same sex. STR analysis following single-cell isolation by DEPArray has been reported for the analysis of Circulating Endothelial Cells enriched from peripheral blood. However single-cell STR analysis on archival samples such as FFPE is hardly achievable due to the DNA degradation hampering single-cell STR analysis.

Non-invasive prenatal screening based on circulating cfDNA for fetal chromosome imbalances can be evaluated for sufficient fetal DNA fraction (FF) since low levels may give rise to false-negative results. Thus, it can be important to estimate the fetal DNA fraction accurately, making sure that it has passed the QC threshold to ensure a sufficient amount of fetal DNA present in a testing sample and make it possible to arrive at a proper interpretation of the sequencing result. Some laboratories may not be assessing FF or not using optimal methods for detection, and this could potentially provide false-negative results to patients. Current approaches developed to estimate fetal DNA fraction using next-generation sequencing include:

- indirect inference of its estimation by evaluating the characteristics of fetal/placental cfDNA differing from that of maternal origin (Cell-Free DNA Size-Based Approach, Cell-Free DNA Nucleosome Track-Based Approach, Fetal Methylation Marker-Based Approach, Shallow-Depth Maternal Plasma DNA Sequencing Data-Based Approach)
- directly assess and quantify genetic variants not present in the maternal background (Y chromosome-based approach, maternal plasma DNA sequencing data with parental genotype-based approach, high-depth sequencing data of maternal plasma DNA-based approach, Shallow-Depth Maternal Plasma DNA Sequencing Data with Maternal Genotype-Based Approach) (Peng XL, Jiang P. Bioinformatics Approaches for Fetal DNA Fraction Estimation in Noninvasive Prenatal Testing. Int J Mol Sci. 2017 Feb 20;18(2) :453).

With maternal plasma DNA sequencing data with parental genotype-based approach (mainly by analysing SNPs), fetal-specific alleles in maternal plasma can be readily identified from the sequence reads. Even though this method is a direct and accurate way to assess the fetal DNA fraction and generally considered as a gold standard, the feasibility of this approach is sometimes hindered by the requirement of parental genotypes, because i) only maternal blood samples would be collected and maternal plasma DNA are subject to sequence for NIPT in most clinical settings; and ii) it is not uncommon that the genotype of the biological father may not be available in practice.

To obviate the requirement of parental genotype information an approach was developed to measure the fetal DNA fraction through the analysis of maternal plasma DNA sequencing data at high depth using targeted massively parallel sequencing. In this method, a binomial mixture model was employed to fit the observed allelic counts with the use of the underlying four types of maternal-fetal genotype combinations and the fetal fraction was determined through the maximum likelihood estimation. The limitation of this approach would be that the sequencing depth is required to be as high as ~120X by targeted sequencing to robustly determine the fetal alleles which impacts on the test cost.

An extended version of this approach was recently developed based on shallow-depth sequencing data coupled with only maternal genotype information (Shallow-Depth Maternal Plasma DNA Sequencing Data with Maternal Genotype-Based Approach). The rationale of this approach is to take advantage of the fact that any alternative allele (non-maternal alleles) present at an SNP locus where the mother is homozygous would theoretically suggest a fetal-specific DNA allele. Thus, the fractions of such non-maternal alleles were hypothesized to correlate with fetal DNA fractions under the assumption that the error rates stemmed from sequencing and genotyping platforms are relatively constant across different cases. However, the parameters in this model might be varied according to sequencing and genotyping platforms, because various platforms are characterized with different error properties, which may contribute to the measured non-maternal alleles. So, it is clear that with Shallow-Depth Maternal Plasma DNA Sequencing and with only homozygous maternal loci (obtained by a SNParray-based genotyping of maternal buffy coat) it is challenging to reliably measure the FF simultaneously with the detection of fetal copy number variations.

Among the closest prior art documents, the following can be cited: Sejoon Lee et al., "NGSCheckMate: software for validating sample identity in next-generation sequencing studies within and across data types", Nucleic Acids Research, 2017, Vol. 45, No. 11, which teaches a method to ensure that NGS datasets from the same subject are properly paired. The NGSCheckMate method, verifies sample identities from FASTQ, BAM or VCF files using a model-based method to compare allele read fractions at approximately 12k or 21k single-nucleotide polymorphisms (SNP) loci, considering depth-dependent behavior of similarity metrics for identical and unrelated samples. NGSCheckMate is effective for a variety of data types, including exome sequencing, whole-genome sequencing, RNAseq, ChIP-seq, targeted sequencing and single-cell whole-genome sequencing, but teaches a requirement for sequencing depth of >0.5X. The requirement is even higher (>3x) in case of kinship or parental relationship samples. In fact, when Sejoon Lee et al. tested their method on a dataset consisting of 89 WGS profiles of single cancer cells from two unrelated glioblastoma patients (39 and 50 cells from each patient), sequenced at a depth (0.01-0.3X) to characterize CNV at the single cell level, they achieved only 87.8% accuracy in grouping the cells, with all misclassification errors due to a few cells with especially shallow sequencing depth (<0.15X).

**Whole genome amplification from single-cells, and low-pass whole genome sequencing**

Whole Genome Amplification (WGA) of single cell genomic DNA is often required for obtaining more DNA in order to simplify and/or allow different types of genetic analyses, including sequencing, SNP detection etc. WGA with a LM-PCR based on a Deterministic Restriction Site (in the following DRS-WGA) is known from WO2000/017390.

DRS-WGA has been shown to be the best-in-class WGA method in many perspectives, in particular in terms of lower allelic drop-out from single cells (Borgstrom et al., 2017; Normand et al., 2016; Babayan et al., 2016; Binder et al., 2014).

A LM-PCR based, DRS-WGA commercial kit (Amplil™ WGA kit, Silicon Biosystems) has been used in Hodgkinson C.L. et al., Nature Medicine 20, 897-903 (2014). In this work, a Copy-Number Analysis by low-pass whole genome sequencing on single-cell WGA material was performed, carrying out digestion of the WGA adaptors and fragmentation prior to Illumina barcoded adaptor ligation for sequencing.

WO2017/178655 and WO2019/016401A1 teach a simplified method to prepare massively parallel sequencing libraries from DRS-WGA (e.g. Ampli1 WGA) for low-pass whole genome sequencing and copy number profiling. In Ferrarini et al., PLoSONE 13(3) :e0193689 https://doi.org/10.1371/journal.pone.0193689, the method performance of WO2017/178655 using the Ion Torrent Platform has been detailed with reference to copy number profiling.

DRS-WGA has been shown to be better than DOP-PCR for the analysis of copy-number profiles from minute amounts of microdissected FFPE material (Stoecklein et al., Am J Pathol. 2002 Jul; 161(1):43-51; Arneson et al., ISRN Oncol. 2012;2012:710692. doi: 10.5402/2012/710692. Epub 2012 Mar 14.), when using array CGH, metaphase CGH, as well as for other genetic analysis assay such as Loss of heterozygosity using targeted primers and PCR for analysis of selected microsatellites, however, it has been shown that depending on FFPE DNA quality, single-cell FFPE LP-WGS is possible but may become impractical for lower DNA quality scores (Mangano, C., Ferrarini, A., Forcato, C. et al. "Precise detection of genomic imbalances at single-cell resolution reveals intra-patient heterogeneity in Hodgkin's lymphoma". Blood Cancer J. 9, 92 (2019). https://doi.org/10.1038/s41408-019-0256-y).

In summary, there is a need to provide a method that allows to infer sample identity and/or analyse the degree of similarity down to single-cell resolution, with low-coverage (< 0.15x) sequencing data, overcoming one or more of the following limitations inherent in the state of the art:

- need of a separate microsatellite analysis assay;
- need of a separate SNP genotyping assay;
- whole-genome sequencing coverage > 0.5x;
- impossibility to reliably reanalyze a single cell for verification or additional targeted genomic information.

For single-cell forensic identification, it would be desirable to have an efficient method, to assign the identity of each of a plurality of single-cell samples even if of poor quality, and further investigate the genetic characteristics of the individual to which said samples belong.

For genome-wide copy number profiling of tumor samples, including single-cell analysis, such as single CTC analysis or single FFPE cells, it may be desirable to provide an inherent sample-tracking algorithm to avoid exchange of low-pass whole genome sequencing samples, and/or detect mix-up of different samples.

For non-invasive prenatal testing or diagnosis on circulating fetal cells harvested from maternal blood, it would be desirable to have an efficient analysis method, combining in a single assay the (i) fetal genome-wide profiling (e.g., genome-wide copy-number profiling) with (ii) the capability to confirm the fetal origin of the sample.

For non-invasive prenatal testing based on circulating fetal cell-free DNA admixed to that of maternal origin using low-pass genome-wide massively parallel sequencing, it would be desirable to have an efficient analysis method that allows i) the identification of the fetal component and the evaluation the its amount in relation to the maternal one (e.g.: fetal fraction, FF) and ii) genome-wide copy-number profiling in the sample from the same low-pass sequencing data.

For pre-implantation genetic screening (PGS; also referred to as pre-implantation genetic testing or "PGT") on e.g. blastocysts, spent embryo culture medium, it would be desirable to have a method using a single assay to detect and/or quantify maternal cell or exogenous contamination in order to avoid false negative or sex discordance calls from the analysis, combining the capability to (i) genome-wide embryo genome profiling (e.g., genome-wide copy-number profiling), which can be used, for example, to confirm presence or absence of aneuploidy in the sample and (ii) quantify and/or determine the absence of maternal contamination, from the same low-pass sequencing data.

For prenatal samples (e.g.: chorionic villi, amniotic fluid, products of conception) it would be desirable to have a method using a single assay to detect and/or quantify maternal cell or exogenous contamination in order to avoid false negative or sex discordance calls from the analysis, combining the capability to i) fetal genome-wide profiling and (ii) quantify and/or determine the absence of maternal contamination, from the same low-pass sequencing data.

In addition to this, it would be desirable to have a method using a single assay to detect in the genetic material derived from the conceptus at any embryo-fetal development phase, conditions such as molar pregnancy, multiple pregnancy (including Vanishing/Chimera), uniparental disomy (isodisomy or heterodisomy) and ROH (Patent n. WO2021019459A1), consanguinity and non-disjunction error classification.

For cell-line authentication, it would be desirable to have a method using a single assay for simultaneous

(i) identification of a cell line using widely available massively parallel sequencers, without the need to run STR analysis on less available capillary electrophoresis instruments, and

(ii) genome-wide profiling (e.g., genome-wide copy number profiling) of the cell line to possibly detect drifts linked to genomic instability or artifacts due to high number of cultural passages.

For FFPE archival samples where single-cell characterization of the individual of origin is desired, such as in analysis regarding endothelial cells in allogenic hematopoietic stem-cell transplantation, it would be desirable to have a technique which can give reliable results from single-cells isolated from FFPE (sorted or microdissected).

<u>Summary</u>

It is therefore an object of the present disclosure to provide a method which overcomes the drawbacks of prior art methods.

In particular, it is an object of the present disclosure to provide a method for analysing the degree of similarity of at least two samples in a plurality of samples comprising genomic DNA, compatible with few cells, down to single-cell, as well as DNA amounts comparable or lower than one genome-equivalent.

This object is achieved by the method as defined in claim 1.

<u>Brief Description of the Drawings</u>

Fig. 1 shows the higher resolution between self and unrelated samples using the method of the present disclosure involving DRS-WGA followed by a fragmentation-free, sequencing-adaptor/WGA fusion-primer PCR reaction, with respect to a random fragmentation library preparation known in the art.

Fig. 2 shows the effect of increasing the number of loci to 300k polymorphic loci based on highest heterozygosity -according to the present disclosure- vs the NGScheckMate selection of 21k SNPs: the discrimination power is increased.

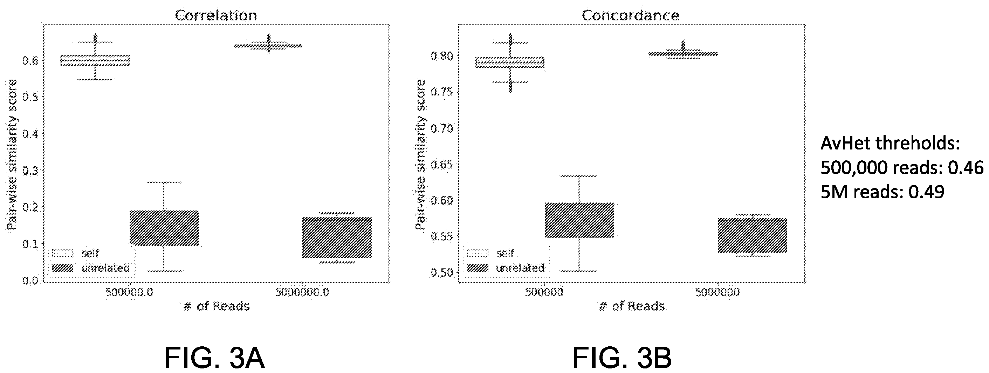

Figs. 3A and 3B show the distribution of similarity scores of paired samples, belonging to the same (self) or different (unrelated) individuals (using cell lines), calculated with different methods according to the present disclosure. In Fig. 3A correlation is used as distance method (standard method of NGSCheckMate). In Fig. 3B concordance is used to assess the similarity of samples. In detail: - if called alleles are the same add 1 to the score; - if called alleles overlap partially (for example if one sample have 2 alleles and the other only 1) add 0.5; - if called alleles are different add 0 to the score. The score is then divided by the number of alleles covered in both samples compared.

Figs. 4A to 4C and 4D to 4F show the relationship between parameters such as minimum average heterozygosity, number of reads and resulting separation between self and unrelated samples.

Figs. 5A to 5D show the performance of classification of kin samples with respect to self female-parent and unrelated samples for a number of reads equal to 500,000 per sample.

Fig. 6 shows the distribution of pair-wise similarity scores calculated as concordance with respect to female-parent samples, for self (female-parent), kin and unrelated samples as a function of minimum average heterozygosity (range = 0.2-0.498).

Fig. 7 shows a sketch of the method to detect twin pregnancies. All pairwise predictions of fetal cells, described by a "kin" relation with maternal control, are used as input to a graph clustering algorithm to find "communities" of fetal cells.

Fig. 8 shows the distribution of average pair-wise similarity scores, calculated with respect to female-parent samples, in erythroblast cell recoveries isolated from peripheral blood of two separate maternal samples.

Figs. 9A to 9C show the clustering based classification of cell recoveries from sample BO1368. The silhouette score of the 2 mixed cells is much lower than that of fetal cells and can be used to discriminate them from fetals and create a new cluster with mixed samples.

Figs. 10A to 10C show the clustering based classification of cell recoveries from sample BO1383.

Figs. 11A and 11B show the performance of classification of individual samples with respect to unrelated samples with at maximum a 50% component of self samples. Fig. 11A is a "ROC-style" plot with TPR and 1-PPV for kin class as a function of "agreement" threshold value. Fig. 11B shows TPR and PPV at different AvHet. The threshold (in grey) has been set in order to have at least a ppv of 99.9%. The threshold is displayed in grey on the secondary y axis.

Fig. 12 shows the distribution of pairwise similarity scores (concordance) calculated for paired samples with various degrees of contamination from a different individual.

Figs. 13A to 13C show the classification of single cell recoveries from FFPE samples according to individuals identity. FFPE samples (lymphoma) from 4 patients. 500,000 reads subsample. Agreement based on concordance. Comparisons have been marked as highDLRS (x axis) if one or both members had a DLRS > 0.4 and lowDLRS if both members had a DLRS $\leq$ 0.4. Fig. 13C shows that clustering correctly assigns all FFPE samples to 4 different clusters corresponding to 4 individuals.

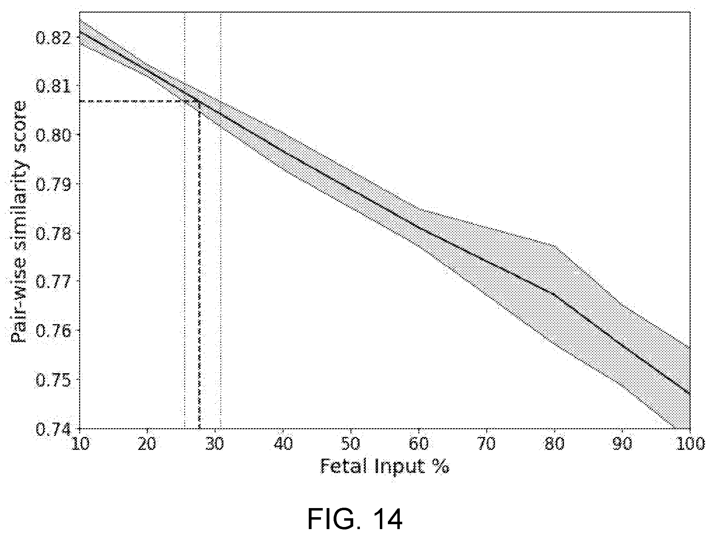

Fig. 14 shows an in-silico simulation of cell-free spent culture media with various degree of maternal DNA contamination from 0 (100% fetal) to 90% (10% fetal), and related similarity score. In particular, the figure shows emulation performed by mixing in silico different proportions of DNA sequences from single fetal cells with sequences from maternal cells. The solid line corresponds to the average pair-wise similarity score at different fetal input percentages. The shaded area corresponds to the 95% confidence interval. Dashed line shows an example of a mixed sample with a known % of maternal component (80%) and a pair-wise similarity score with the maternal reference = 0.807 which, according to the model have a mean predicted fetal component = 27.7% (C.I.= 25.4%-30.7%) corresponding to a estimated contamination from maternal DNA $\approx$ 75%.

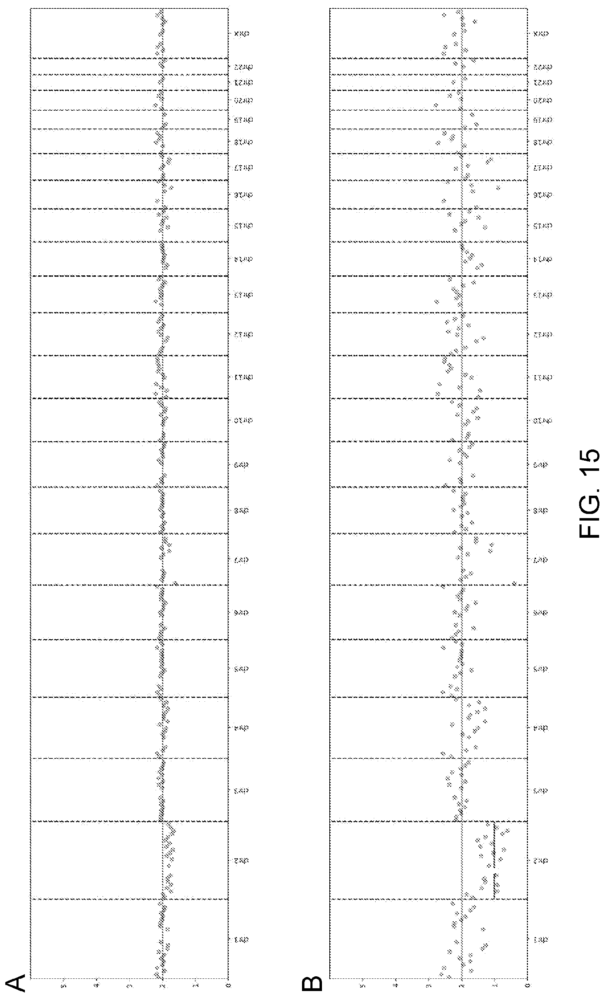

Figs. 15A and 15B show the effect of compensating for contamination in genome-wide copy number analysis of a mixed sample. In particular, the figure represents genome-wide copy number analysis of a mixed sample obtained by in silico mixing of different proportions of DNA sequences from single fetal cells (20%) with sequences from maternal cells (80%). Fig. 15A shows the genome-wide copy number profile; each dot corresponds to a 10Mbp genome bin. Fig. 15B shows the genome-wide copy number after applying a correction factor = 0.75, based on estimated percentage contamination from maternal DNA based on pair-wise similarity score with maternal reference. Statistically significant alterations are shown as solid black lines.

## Definitions

Unless defined otherwise, all technical and scientific terms used herein have the same meaning as commonly understood by one of ordinary skill in the art to which this disclosure pertains. Although many methods and materials similar or equivalent to those described herein may be used in the practice or testing of the present disclosure, preferred methods and materials are described below. Unless mentioned otherwise, the techniques described herein for use with the present disclosure are standard methodologies well known to persons of ordinary skill in the art.

By the expression "massive-parallel next generation sequencing (NGS or MPS)" there is intended a method of sequencing DNA comprising the creation of a library of DNA molecules separated spatially and/or in time, clonally sequenced (with or without prior clonal amplification). Examples include the Illumina platform (Illumina Inc), the Ion Torrent platform (Thermo Fisher Scientific Inc), the Pacific Biosciences platform, the MinIon (Oxford Nanopore Technologies Ltd).

By the expression "low-pass whole genome sequencing" there is intended a whole genome sequencing at mean sequencing depth lower than 1x with reference to the entire Reference Genome, of a massively parallel sequencing library which has not been enriched for sequence-specific fragments. This definition explicitly excludes the case of PCR-based target enrichment or sequence-specific capture-baits target enrichment for a set of loci, such as for example Single-Nucleotide Polymorphisms (SNPs) and/or Short-Tandem Repeats (STR) loci.

By the expression "mean sequencing depth" there is intended here, on a per-sample basis, the total number of bases sequenced, mapped to the reference genome, divided by the total reference genome size. The total number of bases sequenced and mapped can be approximated to the number of mapped reads time the average read length.

By the expression "reference genome" there is intended a reference DNA sequence for the specific species.

By the term "locus" (plural "loci") there is intended a fixed position on a chromosome (relative to the reference genome).

By the expression "polymorphic locus" there is intended a locus having 2 or more alleles with an observed frequency larger than 1% in a population.

By the expression "heterozygous locus" there is intended a locus having 2 or more alleles observed in a specific sample.

By the expression "average heterozygosity" for a locus there is intended the value 1 minus the sum of square of the allelic frequencies. In particular the product 2pq, where p and q=(1-p) are the allelic frequencies for the locus in case of loci with two alleles in the population, or the sum of products 2pq+2pr+2qr, where p, q and r (with p+q+r=1) are the three allelic frequencies for a locus with three possible alleles.

By the expression "covered genome" there is intended the portion of reference genome covered by at least one read.

By the term "read" there is intended the piece of DNA that is sequenced ("read") by the sequencer.

By the expression "reduction ratio" there is intended the total number of bases of fragments, obtained by in-silico digestion of a reference genome according to a restriction enzyme employed in a DRS-WGA, comprised in a specified base-pair range, divided by the total number of bases in the reference genome.

By the expression "allelic content" there is intended the composition in terms of alleles detected at a locus.

By the expression "fragmentation-free, sequencing-adaptor/WGA fusion-primer and PCR reaction" massively-parallel sequencing library preparation, there is intended a massively-parallel sequencing library preparation on DRS-WGA products, without DNA fragmentation steps, whereby sequencing adaptors are added to the WGA product by fusion primers, e.g. according to patent applications (WO2017/178655) or (WO2019/016401A1).

By the expression "pair-wise similarity score", there is intended a function of a plurality of paired inputs with a finite codomain. The codomain is preferably normalized to a standard value, such as [-1;1] or [0;1], independent of the number of paired inputs.

By the expression "sample clustering", there is intended an algorithm for partitioning samples so that samples belonging to the same partition (said also "cluster") share a common property selected from the group consisting of the identity of the one individual (or more individuals) substantially contributing DNA to samples of that partition, the property of containing insufficient quantities of DNA and the property of containing highly degraded DNA or DNA of uncertain origin.

Several metrics of evaluation of the performance of clustering algorithms, when the ground truth is not known, are known in the art such as "Silhouette score", the "Calinski-Harabasz Index", the "Davies-Bouldin Index", which can be used to determine the "optimal" number of clusters for partitioning a plurality of samples into homogenous, well defined clusters.

By the expression "identity-cluster" there is intended a group composed of samples containing with high probability DNA from only one and the same individual. The meaning of high probability (hereinafter Prob[Single-ID]) depends on the application as the skilled in the art would understand and define in relation to the specifics of the application and its performance requirements. For example, in the case of fetal cells analysis, assume that a diagnosis is issued when only at least three single 'putative' fetal cells (i.e. belonging to the identity cluster of cells which are in kin relationship with the maternal reference) are individually analyzed and reported. The diagnosis, e.g. for aneuploidy using the low-pass WGS derived copy-number profile, would be impaired if none of the cells is from an affected fetus, and the cells analyzed are all maternal cells mistaken for fetal. Further assume as acceptable a minimum sensitivity (Sens_min) for the detection of an aneuploid fetus. The ensuing probability of calling normal an aneuploid fetus caused by the miscalling of each of the single-cell identities would require that all cells upon which the diagnosis is based are called fetal instead of maternal. In general it is reasonable to assume these events (pair-wise comparison with the maternal reference) are independent among the putative fetal cells, thus Prob[False_ID of Ncells analyzed] =Prob[False_ID]$^{Ncells}$, were Ncells is the number of cells individually analyzed, where Prob[False_ID]=1-Prob[Single_ID] is the probability of error in calling a sample as belonging to the same-individual identity-cluster (more specifically the cluster of samples in kin relationship with the maternal reference, as said above). One would want that

$(1-Prob[Single\_ID])^{Ncells} \leq (1-Sens\_min)$, i.e.

$Prob\,[Single\_ID] \geq 1-(1-Sens\_min)^{1/Ncells}$

For example, with Sens_min=99.9%, Ncells=5 would require Prob[Single_ID] $\geq$ 75%

while considering Ncells=3 would require

Prob[Single_ID] $\geq$ 90%

In both cases excluding for the sake of simplicity other sources of error like the probability that a truly fetal cell is actually analyzed but fails to detect the aneuploidy.

In the case of forensic investigation and work-up of non-probative samples the meaning of high probability may be different. For example, the method according to the present disclosure may be used to reconstruct an STR profile from a number Ncells of individual cells. Depending on the allowable stringency of the DNA database search, the number of single-cells analyzed, the mean STR call rate for each individual sample from the casework, a different requirement may arise on the exact value of high-probability (Prob[Single_ID]) to meet the objectives.

This requirement is more difficult to model analytically and may be derived for example by Montecarlo simulations by using available databases and simulating *in-silico* various degree of allelic drop-outs, number of single cells actually analyzed, and algorithmic choices in the reconstruction of the profile.

By the expression "single-individual WGA-DNA sample", there is intended a sample comprising a mix of DRS-WGA products obtained from samples containing DNA from a single-individual.

By the expression "non-invasive prenatal testing" there is intended carrying out genetic assays in order to evaluate fetal cell-free DNA or intact fetal cells circulating in maternal blood.

By the expression "pre-implantation genetic testing/screening" there is intended carrying out genetic assays in order to evaluate embryos before transfer to the uterus by genome-wide analysis of, for example, copy-number alterations for determining the presence of aneuploidy (either too many or too few chromosomes) in a developing embryo.

By the expression "pre-implantation genetic diagnosis", there is intended pre-implantation genetic testing by targeted sequencing in order to assay the presence of sequence variants in a developing embryo, such as for example mutations linked to single-gene disorders (e.g., Huntington disease, cystic fibrosis, fragile X syndrome), including those that are autosomal dominant and recessive or X-linked, or hereditary cancer syndromes (e.g., hereditary breast and ovarian cancer, Lynch syndrome). Additionally, this term is intended for sequencing to identify human leukocyte antigen-compatible, unaffected embryos gestated with the goal of allowing ill family members to receive compatible bone marrow transplants or cord blood transfusions.

By the expression "embryonic sample", there is intended a sample containing DNA from an embryo, such as for example a blastocyst, a spent embryo-culture medium, a polar body.

By the expression "single-individual WGA-DNA data", there is intended the data obtained merging sequencing data obtained from samples containing DRS-WGA DNA from a single-individual.

For the sake of simplicity in the description of applications of the method according to the present disclosure in prenatal and reproductive medicine, the term "maternal" may be used to extend its meaning to "belonging to the woman" or "belonging to the female parent", and "mother" to extend to "woman" or "female-parent", with reference to the female individual which has contributed an egg to an embryo, a fetus from an ongoing pregnancy, although that woman may have not become a mother yet as a result of delivering an offspring corresponding to said embryo or fetus etc.

Similarly, the term "paternal" may be used to extend its meaning to "belonging to the man" or "belonging to the male parent", and "father" to extend to "man" or "male-parent", with reference to the male individual which has contributed a sperm to an embryo, a fetus from an ongoing pregnancy, an hydatidiform mole, although that man may have not become a father yet as a result of a woman delivering an offspring corresponding to said embryo or fetus etc.

Detailed Description

The method according to the present disclosure is applied to the analysis of a plurality of samples comprising genomic DNA. In particular, the method is for analyzing the degree of similarity of at least two samples in a plurality of samples comprising genomic DNA. In certain embodiments the samples species is Homo Sapiens, and unless otherwise noted this species will be referred to in the rest of the description, without limitation to the applicability to other species, when applicable.

The method comprises the following steps.

In step a), a plurality of samples comprising genomic DNA are provided.

In step b), a deterministic restriction-site whole genome amplification (DRS-WGA) of said genomic DNA is carried out separately on each sample.

In step c), a massively parallel sequencing library is prepared from each product of said DRS-WGA using a fragmentation-free, sequencing-adaptor/WGA fusion-primer PCR reaction.

In step d), low-pass whole genome sequencing is carried out at a mean coverage depth of < 1x on said massively parallel sequencing library. The mean coverage is preferably 0.01x, preferably at a coverage < 0.05x, more preferably at a coverage < 0.1x, even more preferably at a coverage < 0.5x. This enables a reduction in sequencing costs while maintaining good results in the analysis in relation to the application.

In step e), the reads obtained in step d) are aligned on a reference genome.

In step f), the allelic content at a plurality of polymorphic loci is extracted for each sample, i.e. is obtained from the aligned reads. Said plurality of loci comprises polymorphic loci for the species considered.

Said plurality of polymorphic loci preferably comprises polymorphic loci with average heterozygosity > 0.499, more preferably with average heterozygosity > 0.49, even more preferably with average heterozygosity > 0.4, even more preferably with average heterozygosity > 0.3, the most preferably with average heterozygosity > 0.2.

Said plurality of polymorphic loci preferably comprises > 200,000 loci, more preferably > 300,000 loci, even more preferably > 500,000 loci, the most preferably > 1,000,000 loci.

In step g), a pair-wise similarity score for the at least two samples is calculated, as a function of the allelic content measured at said plurality of loci.

In step h), the degree of similarity of the at least two samples is determined on the basis of the similarity score.

In general, similarity can be measured based on the concordance of allelic content in a shared polymorphic loci, with the word "shared" means the loci is covered by at least one DNA read of the samples in a pair or set of the at least two samples. For example, the pair-wise similarity score is preferably calculated by computing the correlation of the B-allele frequency across loci covered by at least one read in the at least two samples.

As an alternative, the pair-wise similarity score is preferably calculated by computing the mean concordance value across loci covered by at least one read in both paired samples, wherein the concordance value for each locus is assigned one the following values:

a) 1 if the alleles called are identical;
b) 0 if the alleles called are different or completely different;
c) 0.5 if the alleles called are partially overlapping.

For example, in some embodiments, the concordance value for each locus can be assigned:

A1) 1 if the alleles called are identical; and
B1) 0 if the alleles called are different.

Alternatively, in some embodiments, the concordance value for each locus can be assigned:

A2) 1 if the alleles called are identical;
B2) 0 if the alleles called are completely different; and
C2) 0.5 if the alleles called are partially overlapping.

For the purposes of the present disclosure, the methods described herein can be used to couple samples (e.g., single cell samples, cell-free DNA samples, etc) to measure the degree of "similarity" between the samples. The inclusion in the set of samples (i.e., "the at least two samples") of a control sample, such as a maternal/paternal sample in the case of a NIPT assay or paternity testing, respectively, can allow for improved discrimination between samples, such as maternal/paternal and fetal cells.

The method according to the present disclosure preferably further comprises a step of defining a group of clusters of samples sharing a common property such as the identity of the one individual (or more individuals) substantially contributing with DNA to the samples of a cluster, or the property of containing insufficient quantities of DNA and/or the property of containing highly degraded DNA or DNA of uncertain origin.

In another preferred embodiment, a clustering algorithm (e.g. hierarchical clustering) can be implemented to find said clusters using individual samples (e.g., single cells). This type of analysis may be best suited to distinguish groups of samples, where one of the sample is a reference sample used to identify the reference cluster. For example, in NIPT assays, pools of maternal cells can be used as reference to distinguish other groups of cells, such as fetal cells, in pregnant women, using a similarity score as described herein. Clustering approaches in general, and HC specifically, can be implemented including an iterative process for finding the most correct number of clusters, a quality score (e.g. silhouette score) for selecting the best cluster partition, and a way to identify mixed recoveries (e.g. samples belonging to more clusters) and, in the case of NIPT analysis, multiple fetuses.

Preferably, the at least two samples are assigned to at least one cluster by means of a classifier using as input said pair-wise similarity score. As described in further detail below, a classifier may be used independently of clustering analysis.

In a preferred embodiment defining the number of said clusters is carried out by performing an agglomerative clustering of pair-wise similarity score.

In a preferred embodiment such agglomerative clustering is performed using Euclidean distance and ward linkage.

In a preferred embodiment such clustering is performed using a range of numbers of clusters producing different alternative clustering outputs.

In a preferred embodiment such alternative clustering outputs are evaluated by calculating the silhouette score and the clustering with the highest averaged silhouette score across all sub-clusters is selected.

Preferably, said classifier uses as further input at least one value, measured on said low-pass whole-genome sequencing data, selected from the group comprising:

a) DLRS: derivative log ratio spread;

b) R50: percentage of WGA fragments covered by 50% of sequenced reads over total WGA fragments covered by at least one read;

c) YFRAC: fraction of reads mapping to chromosome Y;

d) Aberrant: percentage of genome corresponding to gains or losses respect to median cell ploidy;

e) Chr13: ploidy of chromosome 13;

f) Chr18: ploidy of chromosome 18;

g) Chr21: ploidy of chromosome 21;

h) RSUM: mean absolute deviation from nearest integer copy number level, calculated on the copy number aberration event with highest absolute deviation from median cell ploidy;

i) Mix_score: RSUM z-score, calculated on the copy number aberration event with highest absolute deviation from median cell ploidy; and

j) Deg_score: number of small loss events (< 10 Mbp, which is common in degraded samples).

The number of said clusters is preferably calculated by

a) selecting a number of first-iteration clusters maximizing the average silhouette score;

b) for each one of said first-iteration clusters, computing the silhouette score of each of said samples belonging to the first-iteration cluster, wherein samples belonging to the cluster having a silhouette score lower than a fixed threshold comprised in the range 0.19-0.21, are assigned to a new cluster.

In a preferred embodiment, said group of clusters preferably comprises one or more identity-clusters comprising samples containing, with high confidence, DNA from only one and the same individual.

In the presence of more identity clusters, the cardinality of said plurality of identity-clusters preferably corresponds to the number of individual DNA contributors in said plurality of samples.

Preferably, the method further comprises defining a group of mixed-identity-clusters, each of said mixed-identity clusters comprising samples containing DNA from at least two individuals.

Preferably, the method further comprises defining at least one no-call-cluster, comprising samples containing DNA from uncertain origin.

Advantageously, this cluster includes samples where the number of loci evaluated for calculating the similarity score is lower than a threshold. Advantageously said threshold is established considering one or more elements selected from the group comprising:

1. the number of reads of the sample,

2. the minimum average heterozygosity in the loci used for comparison.

The plurality of samples preferably comprises at least one reference sample and said group of identity clusters includes at least one reference-cluster, comprising said reference sample.

Preferably, a classifier may be used independently of clustering analysis to assign a sample, in a pair, to the correct class using as main input said pair-wise similarity score, and assuming that at least one of the two paired samples is the reference sample. Moreover, a machine-learning classifier may use additional features to obtain the highest possible level of confidence. For the purposes of the present disclosure, it is understood that a classifier does not necessarily assign a sample to a cluster, but rather assigns a sample to one of several predefined classes. Thus, it is possible to classify a sample without clustering it. Conversely, unsupervised clustering techniques can find similarities between samples, without a-priori class definitions.

In a preferred embodiment a machine-learning classifier (e.g. random-forest) can be implemented and trained with a suitable training set to distinguish samples. Such a classifier may use, among other features, the said pair-wise similarity score. This approach may be best suited for pairwise comparisons, where a single test sample needs to be evaluated against a reference sample. An example can be a method in which the goal is to classify a single cell using a pool of cells of known origin as a control (e.g. pool of maternal cells as control). In cell-based non-invasive prenatal testing case, when distinguishing between maternal and fetal cells ), the expected classes may be (i) "self" for the maternal cells, (ii) "kin" for fetal cells, (iii) "mixed" for recoveries comprising a mixture of fetal and maternal cells, (iv) "unrelated" for samples not related to mother or fetus (i.e. exogenous contamination, egg donor in IVF pregnancy, etc.), and "no-call" for unreliable samples with poor metrics. A classifier, such as a random-forest classifier, can distinguish samples using, in addition to the said pairwise similarity score, input from at least one feature measured with low-pass whole-genome sequencing data, including but not limited to:

a) DLRS: derivative log ratio spread;

b) R50: percentage of WGA fragments covered by 50% of sequenced reads over total WGA fragments covered by at least one read;

c) YFRAC: fraction of reads mapping to chromosome Y;

d) Aberrant: percentage of genome corresponding to gains or losses respect to median cell ploidy;

e) Chr13: ploidy of chromosome 13;

f) Chr18: ploidy of chromosome 18;

g) Chr21: ploidy of chromosome 21;

h) RSUM: mean absolute deviation from nearest integer copy number level, calculated on the copy number aberration event with highest absolute deviation from median cell ploidy;

i) Mix_score: RSUM z-score, calculated on the copy number aberration event with highest absolute deviation from median cell ploidy; and

j) Deg_score: number of small loss events (< 10 Mbp, which is common in degraded samples).

Moreover, other types of classifiers that are suitable for the disclosed methods may rely on, for example, a predefined fixed thresholds of said pair-wise similarity score describing the "kin", "self" or "unrelated" relationships (i.e. Example 6).

In some embodiments, clustering strategies (e.g., hierarchical clustering) and classifier strategies (e.g., a RF classifier) may be used interchangeably to distinguish samples based on sequence read data, considering that classifier strategy compares a test sample against a reference sample, while the aim of clustering techniques is to find groups/clusters of samples in which one of these identifies the reference cluster.

In a preferred embodiment, said at least one reference sample is a sample from a pregnant female-parent individual.

Said group of identity-clusters preferably further contains at least one kin-cluster composed by samples from at least one fetus from the ongoing pregnancy of said female-parent individual.

Preferably, said kin-cluster is partitioned in a plurality of fetal-clusters composed of samples which contain DNA from only one and the same fetus.

In an alternative preferred embodiment, said at least one reference cluster is preferably composed by samples containing DNA from only one and same individual corresponding to a victim in a forensic investigation, further comprising defining at least one perpetrator-cluster, comprising samples containing DNA from only one and the same individual, different from a victim.

In this case, the method according to the present disclosure preferably comprises cluster-wise mixing of DRS-WGA aliquots from a plurality of samples belonging to each of said at least one perpetrator-clusters, producing for each cluster a corresponding single-individual WGA-DNA sample, and carrying out further DNA analysis on at least one of said single-individual WGA-DNA samples.

The method preferably comprises cluster-wise merging of genetic analysis data of at least one type of assay, from a plurality of samples belonging to each of said at least one perpetrator-clusters, producing for each of said at least one perpetrator-clusters a corresponding single-individual WGA-DNA data.

The type of assay is selected from the group consisting of microsatellite analysis, single-nucleotide polymorphism analysis, massively parallel targeted sequencing, and whole-genome sequencing.

In one preferred embodiment of the method of the present disclosure, the plurality of samples comprises tumor and/or normal samples.

In another preferred embodiment, the plurality of samples comprises at least a reference sample containing DNA from a female-parent individual, and at least one other embryonic sample from said plurality of samples is selected from the group consisting of:

a) samples containing DNA from an embryo derived from said female-parent individual; and

b) samples containing DNA from a spent embryo-culture medium obtained from an embryo of said female-parent individual.

In the latter embodiment, the method preferably further comprises carrying out a pre-implantation genetic screening on said embryo by analyzing genome-wide chromosomal aberrations from said low-pass whole genome sequencing data from said at least one other embryonic sample using a contamination factor corresponding to maternal contamination measured on said at least one other embryonic sample as a function of said pair-wise similarity of said at least one other embryonic sample from said female-parent individual sample.

In another preferred embodiment, the plurality of samples comprises at least a reference sample containing DNA from a female-parent individual, and at least one other sample containing DNA from a cell-free DNA sample. In some embodiments, the method preferably further comprises carrying out a non-invasive prenatal testing on said cell-free DNA sample by analyzing genome-wide chromosomal aberrations from said low-pass whole genome sequencing data from said at least one cell-free DNA sample using a correction factor corresponding to the fetal fraction measured on said at least one cell-free DNA sample as a function of said pair-wise similarity.

In another preferred embodiment, the plurality of samples comprises at least a reference sample containing DNA from a female-parent individual, and at least one other prenatal sample containing DNA from chorionic villi, amniotic fluid or products of conception. In some embodiments, the method preferably further comprises carrying out a prenatal testing assay on said prenatal samples by analyzing genome-wide chromosomal aberrations from said low-pass whole genome sequencing data from said at least one prenatal sample using a correction factor corresponding to the maternal or exogenous contamination measured on said at least one prenatal sample as a function of said pair-wise similarity.

In particular for cell line authentication, preferably, a plurality of reference clusters is generated from a plurality of samples of DNA from cell lines, and said group of identity clusters further contains at least one samples from a cell line to be authenticated.

In particular for investigating allografts, preferably said at least one reference-cluster is composed by samples containing germline DNA from a transplanted patient, and said group of identity clusters further contains one donor-cluster composed by samples from an allogenic donor of said transplanted patient.

In particular for non-invasive paternity testing, preferably said at least one reference sample comprises a male-parent reference sample containing DNA only from said male-parent, and said at least one reference-cluster further comprises a male-parent identity cluster including said male-parent sample, and:

(i) if the kin-sample similarity score with respect to the male-parent sample is consistent with kinship the paternity is confirmed;
(ii) if kin-sample similarity score with respect to the male-parent sample is consistent with an unrelated individual the paternity is not confirmed.

In particular for non-invasive molar pregnancy assessment, preferably said at least one sample comprises at least one circulating trophoblastic cell sample and, if said trophoblastic cell sample similarity score with respect to the female-parent samples is consistent with unrelated samples, a complete mole is confirmed.

In the latter embodiment, said at least one sample preferably comprises a plurality of trophoblastic cell samples and:

(i) if the similarity score among said trophoblastic cell samples exceeds the expected 99th percentile of the expected similarity score for self samples a P1P1 homozygous paternal mole is confirmed.
(ii) if the similarity score among said trophoblastic cell samples is consistent with the expected similarity score for self samples a P1P2 heterozygous paternal mole is confirmed.

Preferably, said at least one sample further comprises a male-parent sample and the similarity score among said trophoblastic cell samples is consistent with the expected similarity score for self samples, and:

(i) if said trophoblastic cells samples similarity score with respect to the male-parent sample is consistent with the expected similarity score for self samples, a P1P2 heterozygous paternal mole is confirmed.
(ii) if said trophoblastic cells samples similarity score with respect to the male-parent sample is lower than the 1st percentile of the expected similarity score for self samples, a P1P2 heterozygous paternal mole is not confirmed.

By contrast to the state of the art, the inventors surprisingly found that the combination of DRS-WGA with a library preparation for massively parallel sequencing using a fragmentation-free, sequencing-adaptor/WGA fusion-primer PCR reaction for low-pass whole genome sequencing improves the possibility to discriminate DNA samples even from low-pass whole genome sequencing at very shallow depths lower than 1x for self and kin samples, and further also resolve admixed self and kin samples with relatively good accuracy. Moreover, for unrelated individuals, even extremely low coverage whole genome sequencing such as < 0.15x is sufficient.

To prove the above, the following experiments were carried out.

Examples

Example 1

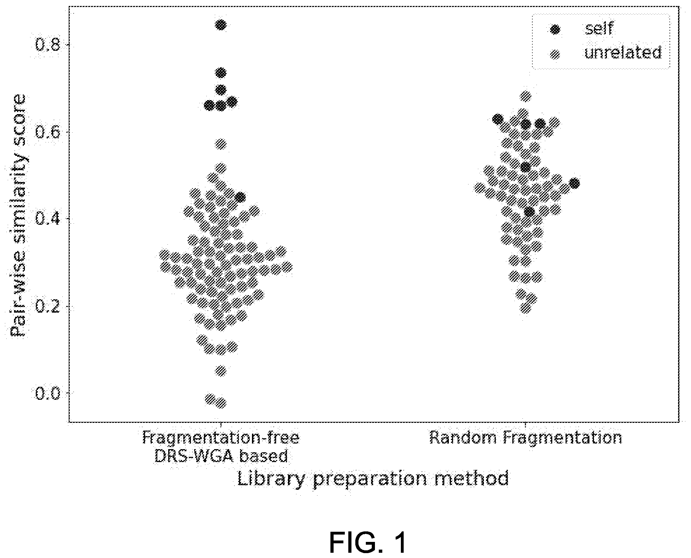

Sequencing data were initially obtained using 7 cell-lines. Fig. 1 shows the effect of the whole genome library preparation method over the correlation of SNP allelic frequencies between self and unrelated samples. On the X axis is the library preparation method. Fragmentation-free libraries have been prepared by performing a deterministic restriction-site whole genome amplification (DRS-WGA) of genomic DNA of 2 single cells of the 7 tumor cell lines (NCI-H1650, NCI-H23, NCI-H661, NCI-H1563, NCI-H1573, NCI-H441, OE19) followed by a fragmentation-free, sequencing-adaptor/WGA fusion-primer PCR reaction; random fragmentation libraries were prepared from genomic DNA of 6 tumor cell lines (NCI-H1650, NCI-H23, NCI-H661, NCI-H1563, NCI-H1573, NCI-H441) using Ion Xpress™ Plus gDNA Fragment Library preparation kit (Thermo Fisher Scientific). On the Y axis is the pair-wise similarity score calculated as the correlation of the B-allele frequency across loci covered by at least one read in the paired samples as reported by NGSCheckMate (commit 8ea2c0438). NGSCheckMate was run on 500,000 reads ($\approx$0.025x coverage) aligned to the reference genome (hg19) with default parameters and default polymorphic loci set (21067 SNPs). Black dots (self) show pair-wise similarity scores of paired samples belonging to the same cell line. Grey dots (unrelated) show pair-wise similarity scores of paired samples belonging to different cell lines. The plot shows a clear advantage of DRS-WGA based fragmentation-free library preparation over Random Fragmentation method with higher separation between self and unrelated pair-wise similarity score values.

Example 2

The polymorphic loci for the comparisons, according to the present disclosure, are preferably selected based on their average heterozygosity. Preferably, polymorphic loci are selected based on the property of having an average heterozygosity higher than a certain minimum threshold.

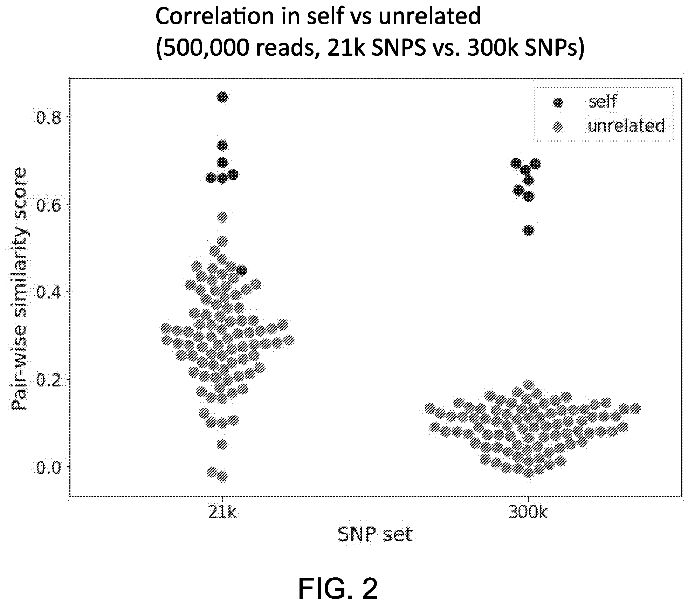

Fig. 2 shows the effect of polymorphic loci set selection on pair-wise similarity scores of paired samples belonging to the same (self) or different cell lines (unrelated). Libraries have been prepared by performing a deterministic restriction-site whole genome amplification (DRS-WGA) of genomic DNA of 2 single cells of 7 tumor cell lines (NCI-H1650, NCI-H23, NCI-H661, NCI-H1563, NCI-H1573, NCI-H441, OE19) followed by a fragmentation-free, sequencing-adaptor/WGA fusion-primer PCR reaction. On the X axis is the polymorphic loci set used for the analysis: 21k set corresponds to default SNP set provided by NGSCheckMate and selected based on allelic frequencies of polymorphic loci in dbSNP in a set of 40 germline WGS profiles from TCGA stomach cancer patients; set 300k consists of 312,458 polymorphic loci selected from dbSNP (build 150) based on a minimum average heterozygosity of 0.498. On the Y axis is the pair-wise similarity score calculated as the correlation of the B-allele frequency across loci covered by at least one read in the at least two samples, the degree of similarity of which is analysed. NGSCheckMate was run on 500,000 reads ($\approx$0.025X coverage) aligned to the reference genome (hg19) with default parameters and either the default polymorphic loci set (21k) or the 300k set. The plot shows that by using a polymorphic loci selection based on average heterozygosity, the difference between pair-wise similarity scores of paired samples belonging to the same cell line (self) and those of paired samples belonging to different cell lines (unrelated) increases leading to a clear separation between the two comparison types.

Different similarity scores calculation methods can be used in step g) according to the present disclosure.

As mentioned in the preceding description, in a preferred embodiment, the pair-wise similarity score of step g) is calculated by computing the correlation of the B-allele frequency across loci covered by at least one read in the at least two samples, the degree of similarity of which is analysed.

In another preferred embodiment, the pair-wise similarity score of step g) is calculated by computing the mean concordance value across loci covered by at least one read in both paired samples, wherein the concordance value for each locus is assigned one the following values:

a) 1 if the alleles called are identical;
b) 0 if the alleles called are completely different;
c) 0.5 if the alleles called are partially overlapping.

Example 3

Figs. 3A and 3B show the pair-wise similarity score distribution computed across samples derived from the same individual ("self") or a different unrelated individual ("unrelated"), for 500,000 reads and minimum average heterozygosity = 0.46 or 5,000,000 reads and minimum average heterozygosity = 0.49, using the correlation (Fig. 3A) or concordance (Fig.3B) methods.

Both methods give similar results in terms of separation and spread of samples from the same class, however the absolute value of the pair-wise similarity score (y-axis) must be clearly changed according to the particular method used. The pair-wise similarity score based on concordance has the advantage of a simpler computation compared to correlation providing a better computational performance, especially in case of large sets of polymorphic loci.

For both read depths the plots show no clear differences in terms of separation of self and unrelated paired samples pair-wise similarity score distributions between the two similarity scores employed, however the absolute value of the similarity score needs to be adjusted for the specific function employed in the calculation.

Example 4 - Average Heterozygosity and number of polymorphic loci

The minimum Average Heterozygosity is preferably in the range [0.2;0.499]. The number of polymorphic loci considered decreases monotonically with increasing minimum Average Heterozygosity.

The number of loci covered by paired samples increases monotonically with the number of reads per sample. There is generally an optimal minimum average heterozygosity for increasing the separation between matched (same individual) and unrelated samples, for a certain number of reads. Further increasing the minimum average heterozygosity beyond that optimum will initially gradually and then suddenly reduce the number of loci covered in paired samples that are available for the comparison, thus reducing the overall separation between matched and unrelated samples in a pair-wise similarity score.

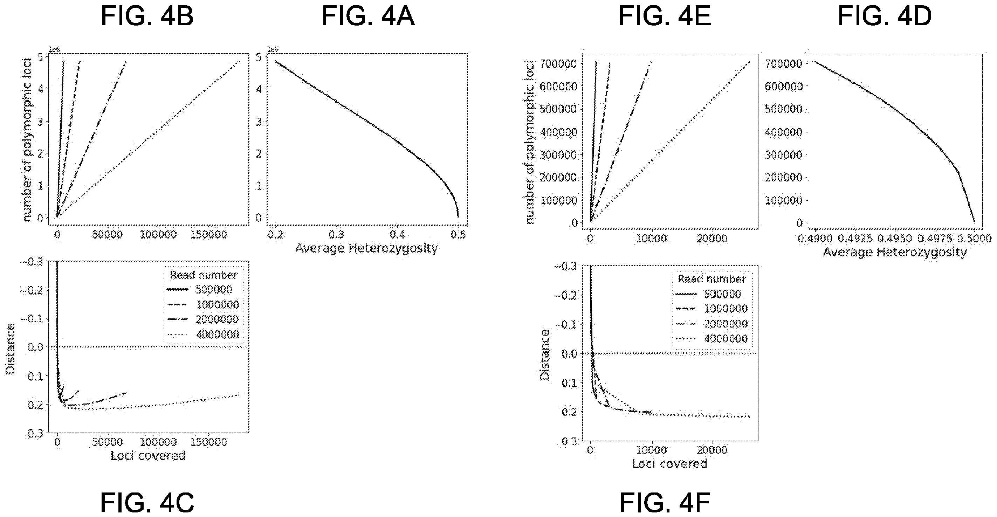

Figs 4A to 4C show the relationship between parameters. Fig. 4A shows the relationship between average heterozygosity threshold (X axis; range = 0.2-0.5) used to select the set of polymorphic loci and number of polymorphic loci (Y axis). Fig. 4B shows the relationship between number of polymorphic loci in the set (Y axis) and average number of loci covered in both paired samples by at least one read (X axis) at different read depths. Fig. 4C shows the relationship between average number of loci covered in both paired samples (X axis) and distance between distribution of pair-wise similarity score (concordance) of paired samples belonging to the same cell line (self) versus that of paired samples belonging to different cell lines (unrelated), calculated as $5^{th}$ percentile of self pair-wise similarity score distribution minus $95^{th}$ percentile of unrelated pair-wise similarity score distribution, at different read depths ranging from 500,000 reads to 4,000,000 reads.

Figs. 4D to 4F are a zoom-in of the same type of analysis for a narrower range of minimum average heterozygosity.

Example 5 - Kinship analysis

An even more difficult problem in sample identification arises in cases of relatedness such as kinship relationship, as for example half of the genome is in common between a mother and her daughter.

In order to evaluate the performance of the method according to the present disclosure in this use case, we simulated this case by generating, *in silico,* kin samples by mixing (50%/50%) low-pass whole genome sequencing data obtained according to the method from single-leukocytes obtained from several (N=3) different unrelated individuals, whereby for each individual, the polymorphic loci were edited in the data so as to report only one of the detected alleles for that individual, thus simulating an haploid genome contribution from that individual to the 'kin' data. From peripheral blood collected in CellSave blood collection tubes (Menarini Silicon Biosystems), following immuno-magnetic enrichment with CELLSEARCH AutoPrep, cells were stained with a cocktail of fluorescent antibodies and DAPI, then CD45+, DAPI+ single-cells were isolated by DEPArray (Menarini Silicon Biosystems), and whole-genome amplified using a DRS-WGA (Ampli1 WGA, Menarini Silicon Biosystems). An aliquot of the WGA product was used to prepare massively parallel sequencing library from each product of those DRS-WGA using a fragmentation-free, sequencing-adaptor/WGA fusion-primer PCR reaction (Ampli1 LowPass kit for Illumina, Menarini Silicon Biosystems).

In order to avoid biases, sequencing data from each single-cell was used only once (either for generating a self or kin data type).

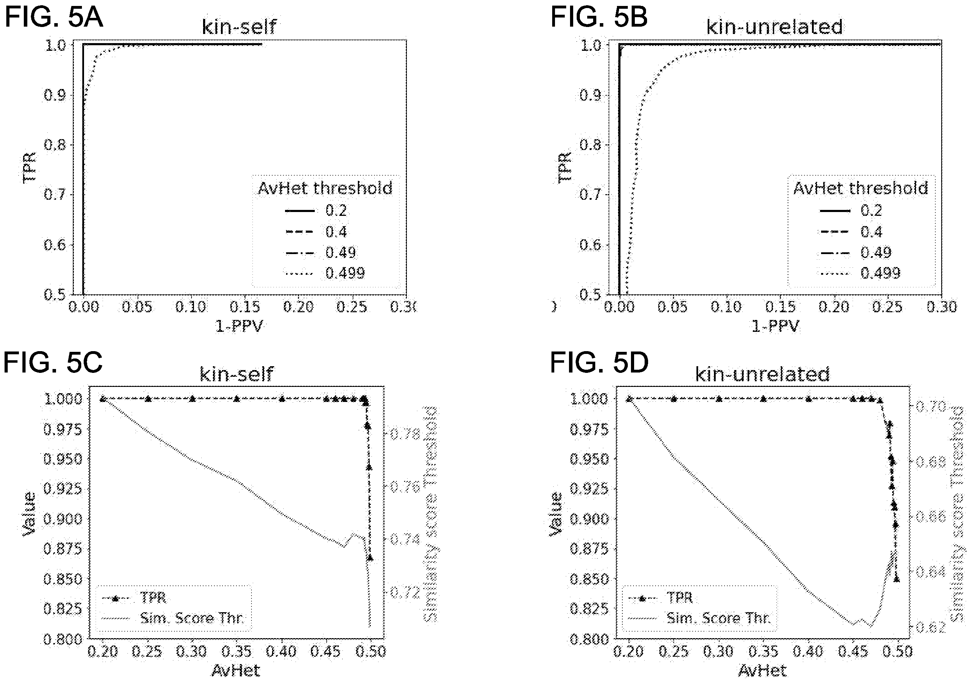

Figs. 5A to 5D show the performance of classification of kin samples with respect to self (female-parent) and unrelated samples. Two variable thresholds on similarity score, calculated with respect to female-parent samples, are used as classifiers to discriminate kin samples from self and unrelated samples. Kin-self threshold is set at values ranging from median of kin similarity score distribution to median of self similarity score distribution. Kin-unrelated threshold is set at values ranging from median of kin similarity score distribution to median of unrelated similarity score distribution. Number of reads is kept constant at 500,000 reads. Fig. 5A shows TPR and 1-PPV values for classification of kin samples with respect to self female-parent as the threshold changes, at different minimum average heterozygosity (AvHet threshold). Fig. 5B shows TPR and 1-PPV values for classification of kin samples with respect to unrelated samples as the threshold changes, at different minimum average heterozygosity (AvHet threshold). Fig. 5C shows kin-self similarity score threshold (grey solid line; secondary Y axis) needed to obtain a PPV of at least 0.999 and corresponding TPR (primary Y axis) as the value of minimum average heterozygosity changes (X axis). Fig. 5D shows kin-self similarity score threshold (grey solid line; secondary Y axis) needed to obtain a PPV of at least 0.999 and corresponding TPR (primary Y axis) as the value of minimum average heterozygosity changes (X axis). The plots show that a high sensitivity (TPR $\geq$ 0.99) is obtained with SNP sets selected using an average heterozygosity threshold from 0.2 up to 0.495 for kin-self classification and up to 0.48 for kin-unrelated classification with sensitivity values decreasing rapidly past these values.

Example 6

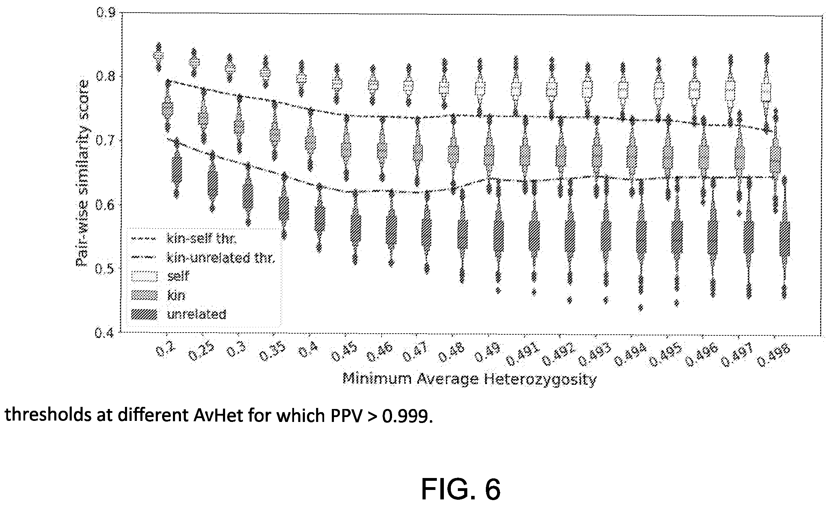

Fig. 6 shows the distribution of pair-wise similarity scores calculated as concordance with respect to female-parent samples, for self (female-parent), kin and unrelated samples as a function of minimum average heterozygosity (range = 0.2-0.498). Number of reads is kept constant at 500,000 reads. Similarity score thresholds used to classify kin samples from self female-parent samples and unrelated samples with PPV of at least 0.999 are shown as dashed and dot-dashed lines respectively.

Accordingly, in a preferred embodiment, the LPWGS data is subsampled to 500k single reads, the minimum average heterozygosity for polymorphic loci is selected in the range [0.2;0.49] and the similarity score thresholds are selected in the range [0.73;0.79] for kin-self and [0.62;0.7] for kin-unrelated, using as similarity score "concordance" calculated as explained above. The plurality of polymorphic loci preferably comprises loci obtained from a database, such as dbSNP. Preferably said plurality of polymorphic loci comprises > 200.000, 300.000, 500.000 or 1.000.000 loci with highest average heterozygosity.

**Clustering**

In a preferred embodiment, the method according to the present disclosure further comprises a step of defining a group of clusters of samples sharing a common property such as the identity of the one individual (or more individuals) substantially contributing with DNA to the samples of a cluster, or the property of containing insufficient quantities of DNA and/or the property of containing highly degraded DNA or DNA of uncertain origin. The at least two samples are preferably assigned to at least one cluster by means of a classifier using said similarity score and other quality metrics.

Example 7 - Application to non-invasive prenatal diagnosis based on fetal circulating cells.

In a preferred embodiment, the at least one reference-cluster is composed of samples from a pregnant female-parent individual. Said "reference samples" may be collected isolating maternal cells from the same enriched bodily fluid used to extract fetal cells, or alternatively by another source of maternal DNA. In case the maternal bodily fluid consists of peripheral blood, nucleated cells positive for maternal markers and negative for fetal markers can be collected as reference.

Preferably said group of identity-clusters may further contain at least one kin-cluster composed by samples from at least one fetus from the ongoing pregnancy of said female-parent individual. Said samples are identified preferably as those having a pair-wise similarity score consistent with a kin-relationship with the reference female-parent.

Said kin-cluster is preferably further partitioned in a plurality of fetal-clusters composed of samples which contain DNA from only one and the same fetus.

Samples belonging to the same fetus are recognized as having a pair-wise distance score consistent with a classification as self with respect to each-other. Other kin cells having a pair-wise distance score consistent with a kin relationship with respect to other kin cells are put in a different partition as belonging to a different fetus.

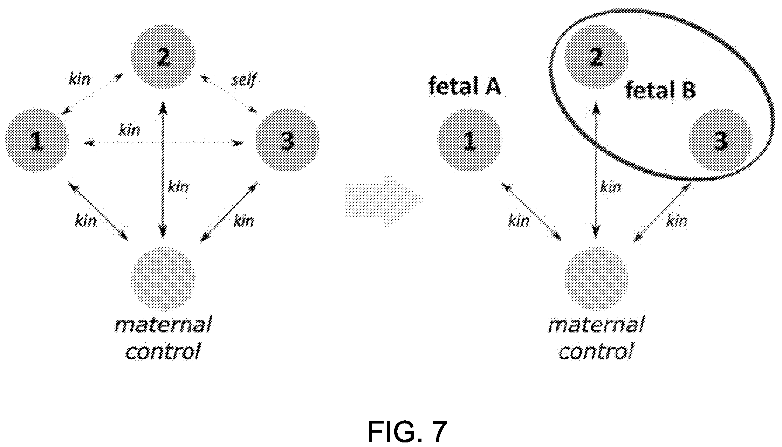

Fig. 7 represents a method to detect twin pregnancies. All pairwise predictions of fetal cells, described by a "kin" relation with maternal control, are used as input to a graph clustering algorithm to find "communities" of fetal cells.

In another embodiment useful in the context of Non-Invasive Prenatal Diagnosis, circulating fetal cells admixed to maternal cells are detected by observing a pair-wise similarity score intermediate with respect to that expected for "self" type DNA and "kin" type DNA. In fact, the co-isolation of a maternal cell along with a target fetal cell may accidentally occur as a result of imprecision in the sorting process (either due to the selection of cells to isolate or due to the isolation process, or both). Co-isolation of a maternal cell along with a target fetal cell may also occur non-accidentally, as it may be beneficial to anyway analyze an additional mixed sample instead of discarding it, if too few non-admixed and pure fetal cell samples are available.

Depending on the type of analysis, the admixture of two cells, one fetal and one maternal, may still be acceptable, if the sensitivity of the assay is not significantly impaired. This can be for example the case when analyzing whole chromosome aneuploidies, using adequate numbers of reads. The contamination may be advantageously factored-in during the analysis by applying a specific contamination factor, as it is available in certain bioinformatic pipelines, such as ControlFreec (Boeva, V. et al, Bioinformatics 2012 Feb 1;28(3):423-5), thus maintaining an adequate sensitivity.

In a preferred embodiment, said fetal cells circulating in maternal blood are (i) trophoblasts, (ii) erythroblasts or (iii) both types.

Example 8 - Identification of circulating fetal erythroblasts from maternal blood.

Nucleated cells were first isolated from maternal blood using a ficoll gradient (density 1.107g/ml), and fetal erythroblasts (nucleated red blood cells) were enriched by CD45/CD15/CD14 immuno-magnetic depletion of unwanted maternal cells using Magnetic Activated Cell Sorting (MACS) from Miltenyi.

The enriched cells were fixed, with either

(A) Paraformaldehyde (PFA) 4% for 30' at Room Temperature, or

(B) PFA 4% 60' 37° followed by 0.05% Glutaraldheide for 30" at Room Temperature

The second type of fixation, creates stronger crosslinking and may help fixing the target hemoglobin within the cell, however hampers the DNA amplification.

After fixation, cells were stained for anti-gamma-Hemoglobin-FITC (as a fetal cell marker) and DAPI to stain the DNA in the nuclei.

Putative fetal cells were sorted by DEPArray™ as single-cells, or along with additional maternal contaminating cells which happened to be co-located in the same dielectrophoretic cage. Cell recoveries (regardless if single or contaminated) were amplified with Ampli1 WGA kit, Menarini Silicon Biosystems S.p.A., a kit implementing the DRS-WGA method according to the present disclosure.

An aliquot (1ul) of the Ampli1 WGA primary PCR product was used for Microsatellite analysis, with a multiplex PCR for amplifying the following loci: D21S1435, D21S11, HPRT, SRY, D21S1413, D21S1411, D18S535, D13S317, D21S2039, D13S631, D21S1442, followed by fragment analysis using capillary electrophoresis on ABI Prism 310 (Applied Biosystems). Using the "weaker' fixation protocol -option (A) above- 56% of the expected alleles were recovered on average (range 30%-90%). On average 3.2 informative alleles were found, defined as alleles not in common between mother and fetal reference profile obtained by analysis of the Chorionic Villi Sample (CVS).

Using the 'stronger' fixation protocol -option (B) above- only 28% of the expected alleles were recovered on average (range 6%-68%), i.e. about half of those recovered with weaker fixation. In other terms, with *stronger* fixation (B), an average allelic drop-out of 72% was obtained. Correspondingly, on average only 1.7 informative alleles were found, including also mixed samples (BO1368B_4, BO1368B_6) having both maternal and fetal informative alleles, thus having two cells and double the amount of starting DNA template. Indeed, 4 single-cell samples (BO1368B_3, BO1368B_5, BO1368B_9, BO1368B_12) had 0 informative alleles on the above STR multiplex analysis. The first three of them were only resolved with additional analysis using further STR loci, analysis which failed to provide information to classify sample BO1368B_12, which remained of "Unknown" origin.

It is thus clear that, while it provides more fetal erythroblasts, Stronger fixation (such as PFA 4% 60' 37° followed by 0.05% Glutaraldehyde for 30" at Room Temperature) increases allelic drop-outs and reduces STR call rate, thus severely jeopardizing classification of a sample as maternal, fetal, or mixed.

Conversely, preparing from another aliquot of WGA product a massively sequencing library using Ampli1 LowPass kit, and analyzing the data using the method according to the present disclosure it is possible to confidently assign each sample, as further described in more detail in what follows, even for such samples with very high allelic dropout.

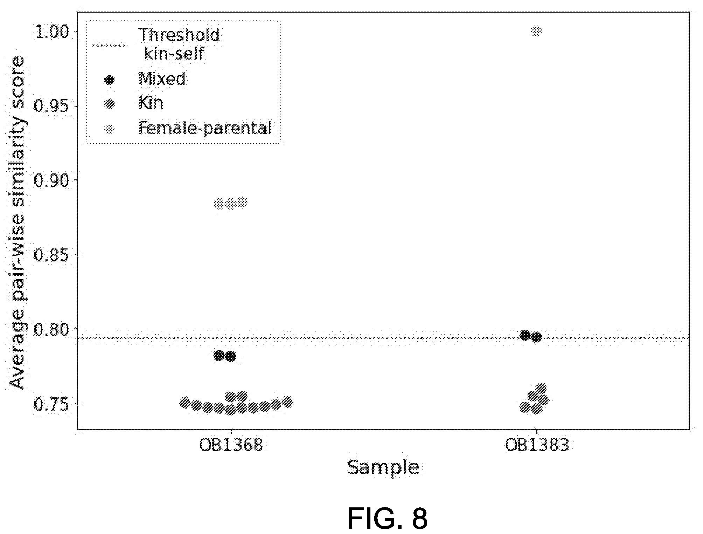

Fig. 8 shows the distribution of average pair-wise similarity scores, calculated with respect to female-parent samples, in erythroblast cell recoveries from 2 samples. The plot shows that the kin-self threshold classifier discriminates kin recoveries (grey dots) from pregnant female-parent individual cell recoveries (light grey dots). However the classifier cannot discriminate kin recoveries from mixed cell recoveries (black dots).

In a preferred embodiment, the clustering of samples includes computing a silhouette-score, based on the similarity, in order to define the number of clusters. Advantageously, a cluster where the pair-wise similarity scores display two distinct level of similarity can be further fractionated by using a fixed threshold, preferably 0,205, based on the distribution of silhouette scores in a set of samples comprising maternal cells and fetal cells, to discriminate mixed fetal-maternal samples (from fetal or maternal samples). In a preferred embodiment, said fixed threshold is within the range [0.19-0.21].

In this way, mixed maternal-fetal cells can be identified as a separate cluster from the self (maternal) and kin (fetal) subpopulation.

Example 9

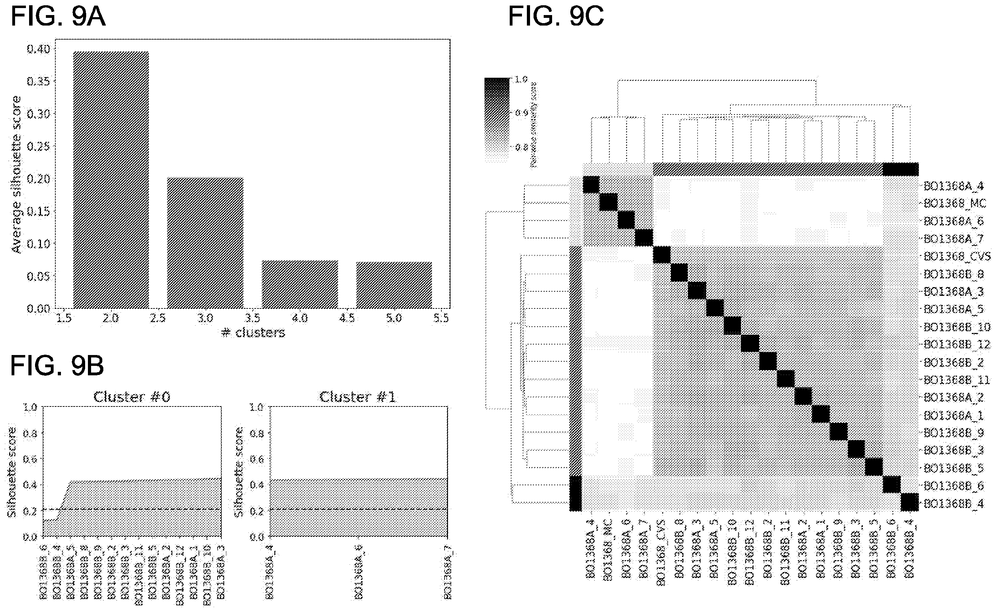

Figs. 9A to 9C show the clustering based classification of cell recoveries from sample BO1368. A maternal cells sample (BO1368_MC) and a chorionic villus sampling (BO1368_CVS) are included as reference. Fig.9A shows the average silhouette scores for different numbers of clusters, used as input for clustering of pair-wise similarity scores, showing the highest score for 2 clusters. Fig. 9B shows the analysis of individual silhouette score for each recovery in the two clusters shows that 2 recoveries in cluster #0, corresponding to mixed cell recoveries, have a score close to 0 indicating that they are very close to the decision boundary between two neighboring clusters; by setting a fixed minimum silhouette score threshold (0.205) it is possible to discriminate the 2 mixed fetal-maternal cell recoveries which are thus assigned to a third independent cluster. Fig. 9C shows the heatmap showing similarity scores between all 17 cell recoveries in shades of grey with darker colors indicating higher similarity; clusters are labeled by row and column color labels.

Example 10

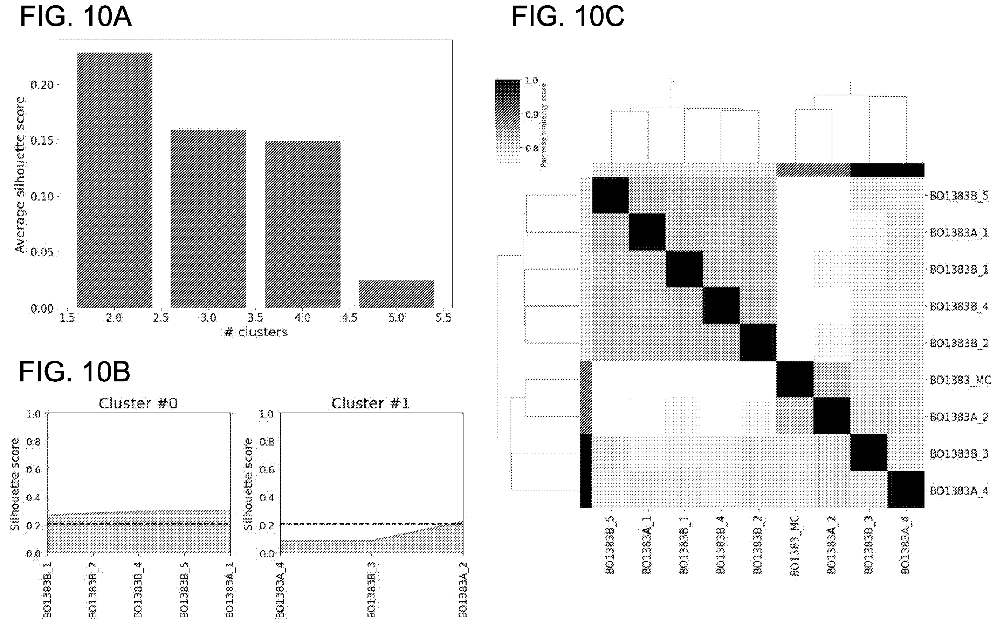

Figs. 10A to 10C show the clustering based classification of cell recoveries from sample BO1383. A maternal cell sample (BO1383_MC) is included as reference. Fig. 10A shows the average silhouette scores for different numbers of clusters, used as input for clustering of pair-wise similarity scores, showing the highest score for 2 clusters. Fig. 10B shows the analysis of individual silhouette score for each recovery in the two clusters shows that 2 recoveries in cluster #0, corresponding to mixed cell recoveries, have a score close to 0 indicating that they are very close to the decision boundary between two neighboring clusters; by setting a fixed minimum silhouette score threshold (0.205) it is possible to discriminate the 2 mixed fetal-maternal cell recoveries which are thus assigned to a third independent cluster. Fig. 10C shows the heatmap showing similarity scores between all 8 cell recoveries in shades of grey with darker colors indicating higher similarity; clusters are labeled by row and column color labels.

Example 11 - Application to non-invasive prenatal paternity testing based on fetal circulating cells.

In another embodiment of the present disclosure, a male-parent sample (paternal sample) is available in addition to the maternal sample, and the kinship analysis may be applied using in turn as reference also the paternal sample. A pair-wise similarity score consistent with a "kin" type DNA with respect to the paternal reference sample confirms the paternity of the fetus. Alternatively, if a pair-wise similarity score of the fetal sample (i.e. confirmed fetal because classified as kin with respect to the female-parent reference sample) is consistent with an "unrelated" type DNA using the male-parent samples, the result confutes the paternity.

Example 12 - Application to molar pregnancy.

In another embodiment of the present disclosure, at least one putative circulating fetal trophoblastic cell is enriched from the maternal blood. The trophoblastic cell sample is compared to the maternal reference sample, and a pair-wise similarity score consistent with an "unrelated" type DNA indicates a possible complete mole (or a lab contamination/sample swapping). If more than one sample of circulating trophoblastic cells is isolated, comparison of the pair-wise similarity score among those samples can be used to study the genotype of the mole. If the pairwise distance largely exceeds the expected value for paired samples of type "self", a P1P1 homozygous paternal mole is confirmed, as all the comparison of the polymorphic loci will be identical, except for rare sequencing errors (or even more rare WGA amplification errors) which may occasionally occur in the same genomic positions corresponding to the polymorphic loci examined. Alternatively, in presence of a P1P2 mole with heterozygosity in some of the polymorphic loci, the pairwise similarity value observed among different trophoblast samples is in the range expected for paired samples of type "self". In this latter P1P2 mole case, if a paternal DNA sample is available, a pair-wise distance score of the trophoblast samples consistent with a "self" type DNA with respect to the paternal reference sample may be used to distinguish the molar pregnancy from a lab contamination or sample swap.

Example 13 - Application to single-cell forensic and human identification.

In a preferred embodiment, said at least one reference cluster is composed by samples containing DNA from only one and same individual corresponding to a victim in a forensic investigation, further comprising defining at least one perpetrator-cluster, comprising samples containing DNA from only one and the same individual, different from a victim.
Samples are assigned to a perpetrator cluster if they have a pair-wise distance score consistent with an "unrelated" relationship with the victim samples, and a "self" relationship with other samples belonging to the same perpetrator cluster. Whenever a new sample is consistent with "unrelated" to both the victim and perpetrators already belonging to other perpetrator-clusters, a new perpetrator-cluster is defined.
Alternatively, the use of a clustering algorithm based on silhouette-score, as detailed for the case of non-invasive prenatal diagnosis application, can be used to assign each individual sample to an homogenous cluster.
Advantageously, in case of forensic identification, samples with a pair-wise distance score consistent with a "kin" relationship (as obtained with the non-invasive prenatal diagnosis -NIPD- type of analysis) may be interpreted as "mixed samples", as they likely contain DNA from two unrelated individuals (victim and perpetrator, or different perpetrators), similar to the case of "kin" samples in NIPD application, which contains DNA from one female-parent and one unrelated male-parent.
Advantageously, copy-number information for sex chromosomes, obtained by the same low-pass whole genome sequencing data, may be used to further refine and/or confirm the classification based on a refined pair-wise distance score.
In case of sex-mismatch between victim and perpetrators, as it is common in sexual-assault evidence, the copy-number information on chromosome X and Y can help inform sample classification as victim or perpetrator.

In another preferred embodiment, said at least one reference cluster is composed by samples containing DNA from only one and same individual corresponding to a suspect perpetrator in a forensic investigation, further comprising defining at least one perpetrator-cluster, comprising samples containing DNA from only one and the same individual.

In another preferred embodiment, a plurality of samples obtained by a mixed forensic evidence with multiple DNA contributors, each sample containing one or more cells, are analyzed according to the method, further comprising defining at least one perpetrator-cluster, comprising samples containing DNA from only one and the same individual.

In a preferred embodiment, DRS-WGA aliquots from a plurality of samples belonging each to the same of said at least one perpetrator-clusters, are mixed together, thus producing for each cluster a corresponding single-individual WGA-DNA sample, thus enabling one to carry out further DNA analysis on said single-individual WGA-DNA sample. The advantage of this approach is that potential random allelic drop-outs occurring in a single-cell sample are complemented by the signal from other individual cells, thus producing a more complete profile. This approach is particularly advantageous when the DNA of each single-cell sample from an individual is strongly degraded. This may happen in particular for cold cases, especially when the evidence has been stored at room temperature, or cases where a tissue sample from the victim has been fixed in formalin and embedded in paraffin for later use.

Another preferred embodiment, comprises cluster-wise merging of genetic analysis data of at least one type of assay, from a plurality of samples belonging to each of said at least one perpetrator-clusters, producing for each of said at least one perpetrator-clusters a corresponding single-individual WGA-DNA data.

In a preferred embodiment, said at least one type of assay is selected from the group consisting of:

a) microsatellite analysis;
b) single-nucleotide polymorphism analysis;
c) massively parallel targeted sequencing;
d) whole-genome sequencing.

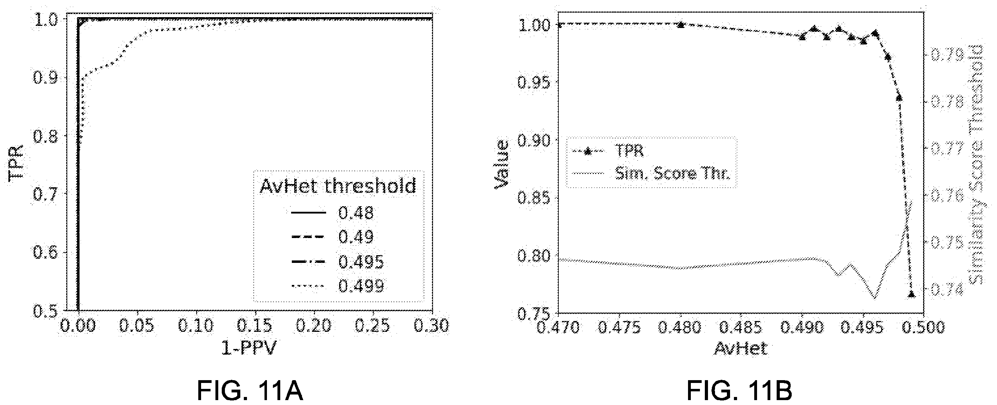

Figs. 11A and 11B show the performance of classification of individual samples with respect to unrelated samples with at maximum a 50% component of self samples. A classifier based on a variable threshold on pair-wise similarity score is used to discriminate samples from an individual from mixed samples. The threshold is set at values ranging from median of "self" similarity score distribution to median of "mixed" similarity score distribution. Number of reads is kept constant at 500,000 reads. A) TPR and 1-PPV values for the classifier as the threshold changes, at different average heterozygosity (AvHet threshold). B) Pair-wise similarity score threshold (grey solid line; secondary Y axis) needed to obtain a PPV of at least 0.999 and corresponding TPR (primary Y axis) as a function of the average heterozygosity (X axis) . The plots show that a high sensitivity (TPR $\geq$ 0.99) is obtained with SNP sets selected using an average heterozygosity threshold from 0.2 up to 0.495 for kin-self classification and up to 0.48 for self-mixed classification with sensitivity values decreasing rapidly past these values.

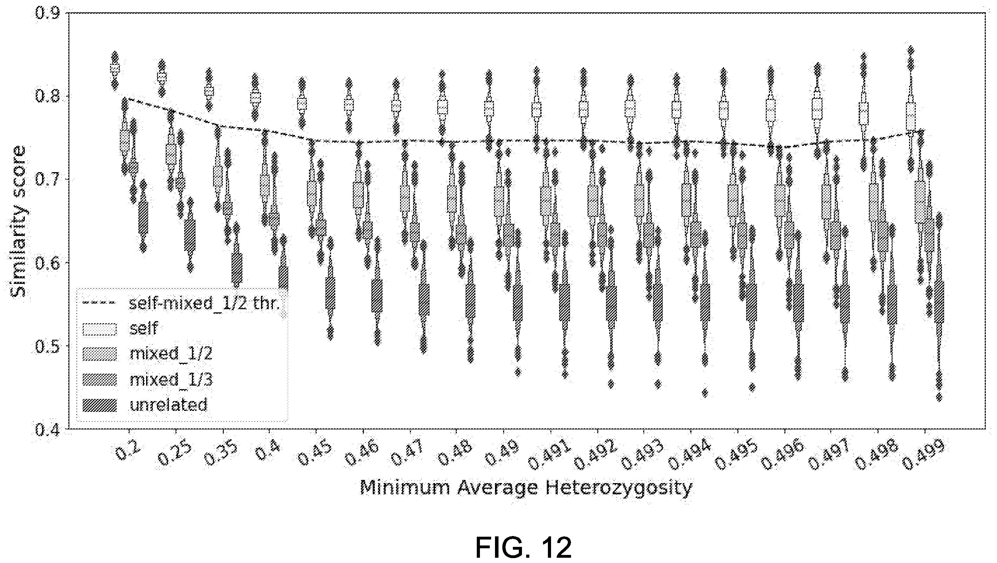

Fig. 12 shows the distribution of pairwise similarity scores (concordance) calculated for paired samples from the same individual (self), for paired samples where one of the samples contains a 50% component from the same individual as the other sample (mixed_1/2), for paired samples where one of the samples contains 1/3 (33%) of the same individual as 'self' and a 66% component from the same individual as the other sample (mixed_1/3), for paired sample belonging to different individuals (unrelated), as a function of the average heterozygosity (range = 0.2-0.499). Number of reads is kept constant at 500,000 reads. Classifier based on pair-wise similarity score shown as dashed line.

The term perpetrator and victim used above are to be intended just as guidance and help in the comprehension. It is clear to those with ordinary skills in the art that the above method is applicable, without departing from the present disclosure, also to other settings of human identification, such as the identification of individuals victims of a disaster, where the cluster meaning is just recast from perpetrator to a different arbitrary name.

Example 14 - Application sample identification in oncology laboratory workflow.

In another preferred embodiment, the method according to the present disclosure is used to match samples belonging to the same patient and detecting both possible sample swaps, or possible cross-contaminations from samples belonging to different patients. For example, this may be particularly beneficial when working with single-cell FFPE samples. In fact, it is utterly difficult to get exhaustive genomic information from single-cell (or nuclei) extracted from FFPE due to the DNA damage caused by the fixation. STR or even targeted sequencing for SNPs may be impractical. However using the method according to the present disclosure it is still possible to distinguish the samples.

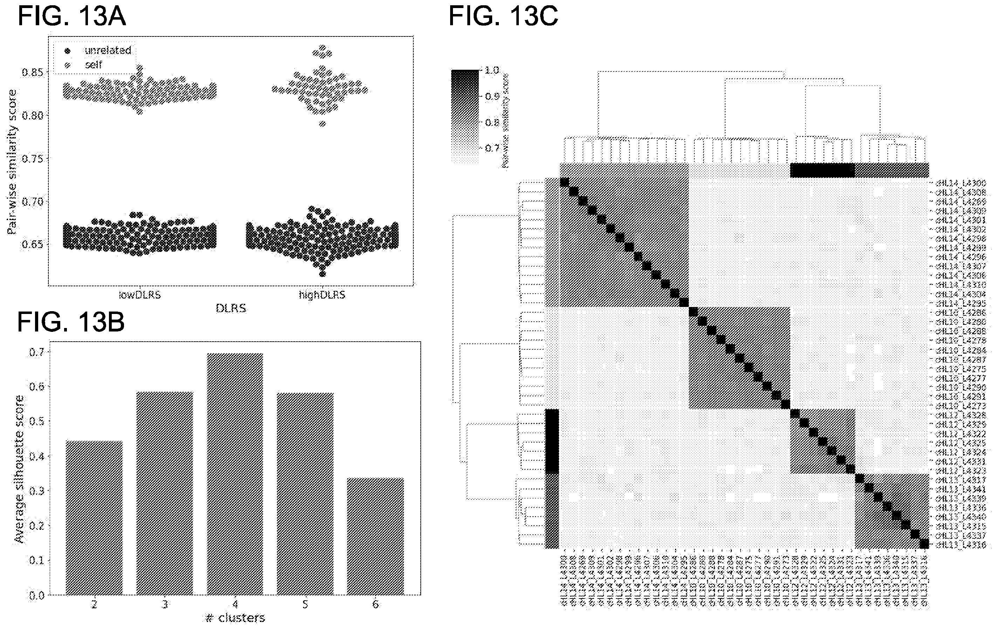

Figs. 13A to 13C show the classification of single cell recoveries from FFPE samples according to individuals identity. The single-cell WGA products were obtained as detailed in Mangano C. et al., "Precise detection of genomic imbalances at single-cell resolution reveals intra-patient heterogeneity in Hodgkin's lymphoma", Blood Cancer Journal volume 9, Article number: 92 (2019). Fig. 13A shows a Swarmplot showing pair-wise similarity scores of paired samples belonging to the same individual (self) or to different individuals (unrelated). Data are binned according to genome-wide copy number signal DLRS (X axis), where lowDLRS corresponds to paired samples with DLRS < 0.4, indicative of low signal noise and highDLRS corresponds to paired samples where at least one of the samples in the pair shows a DLRS $\geq$ 0.4, indicative of high signal noise. For both bins the plots show a clear separation, in terms of pair-wise similarity score, between self and unrelated samples. Fig. 13B shows the average silhouette scores for different numbers of clusters, used as input for KMeans clustering of pair-wise similarity scores, showing the highest score for 4 clusters. Fig. 13C shows the heatmap showing pair-wise similarity scores between all 17 cell recoveries in shades of grey with darker colors indicating higher similarity; clusters are labeled by row and column color labels; for visualization purposes rows and columns are ordered by euclidean-distance based hierarchical clustering.

Example 15 - Application of sample identification in pre-implantation genetic screening (PGS).

In another preferred embodiment, the method according to the present disclosure is used to analyze samples deriving from a cell-free spent embryo-culture medium. As known in the art, it is beneficial to assess embryos to prioritize for implantation in order to increase the uptake rate and success of the procedure. Techniques based on cell-free spent culture medium are attractive as they simplify the workflow and may be less invasive for the developing embryo. However contamination from maternal DNA has been reported in the culture medium and shown to impair the resolution of the PGS in detecting aneuploidies in the fetus.

In an embodiment of the present disclosure in this application context, the maternal reference is used as a reference for "self" (female-parent). The pairwise similarity score with the cell-free spent embryo-culture medium sample is computed according to the present disclosure. Said pair-wise similarity score is used to estimate the contamination from maternal DNA with respect to the embryo DNA. Pair-wise similarity score lower or equal to the expected median value for a "kin"-type DNA with respect to the maternal reference is used to assume 100% purity of embryonic DNA. Pair-wise similarity score equal or higher to the expected median value for a "self"-type DNA with respect to the maternal reference is used to assume 0% purity of embryonic DNA (all maternal DNA) in the cell-free sample. An intermediate value of pair-wise similarity indicates a degree of contamination from maternal DNA. This contamination value can be used as input in the genome-wide copy number profiling analysis based on the same low-pass whole genome sequencing data, in order to compensate for the potential dilution -due to the admixed signal deriving from the normal diploid maternal genome- of the copy-number signal stemming from potential aneuploidy or sub-chromosomal copy-number variations of the embryo. In this way, due to the compensation, the sensitivity of the copy-number caller is less impaired by the signal dilution. In addition, the contamination value can be used to assess the suitability of the sample to detect reliably copy-number variations of a given size, as the degree of diploid maternal background can impair the detection of sub-chromosomal CNVs, e.g. microdeletions.

Fig. 14 shows a simulation performed by mixing, *in silico,* different proportions of DNA sequences from single fetal cells with sequences from maternal cells. The solid line corresponds to the average pair-wise similarity score at different fetal input percentages. The shaded area corresponds to the 95% confidence interval. Dashed line shows an example of a mixed sample with a known % of maternal component (80%) and a pair-wise similarity score with the maternal reference = 0.807 which, according to the model have a mean predicted fetal component = 27.7% (C.I.= 25.4%-30.7%) corresponding to an estimated contamination from maternal DNA $\approx$ 750.

Figs. 15A and 15B show the genome-wide copy number analysis of a mixed sample obtained by in silico mixing of different proportions of DNA sequences from single fetal cells (20%) with sequences from maternal cells (80%). Fig. 15A shows a genome-wide copy number profile; each dot corresponds to a 10Mbp genome bin. Fig. 15B shows a genome-wide copy number after applying a correction factor = 0.75, based on estimated percentage contamination from maternal DNA based on pair-wise similarity score with maternal reference. Statistically significant alterations are shown as solid black lines.

A similar approach can be used also for cell-free DNA or invasive prenatal samples to determine the fetal fraction and contamination, respectively, using a reference comprising plasma leucocytes for cell-free DNA, maternal decidua, buccal swab, or blood.

Example 16 - Application to sample identification in cell-line authentication.

In another preferred embodiment, the method according to the present disclosure is used to authenticate cell lines used in research laboratories.

In this embodiment, a *reference* database collecting - from all *reference* cell-line types- the base-line low-pass WGS data according to the method is first established, so that data from this reference database is used to authenticate the cell line *under-test.*

In a preferred embodiment for this application, the starting samples are preferably selected from the group composed of (i) a pool of cells or (ii) DNA extracted from a pool of cells.

In this way:

- for the *reference* sample of pure cell lines an average comprehensive profile of the cell-line is obtained, best summarizing the diversity linked to cell heterogeneity;
- for the sample *under-test,* in addition, a potential contamination from another cell line may be observed. A threshold based on the distribution of similarity scores among repetitions of the assay may be used to call a contamination, with a certain degree of confidence, if the similarity score is lower than that minimum threshold. In addition, using an approach similar to what reported above for the application to pre-implantation genetic screening, an indirect measure of the amount of the contamination may be obtained, comparing the observed similarity score of sample under-test to a calibration curve representing the expected similarity score as a function of the contamination of a pure 'self' by another generic 'unrelated' sample.

The number of cells in said pool is preferably in the range [50-1.500]. The lower limit of 50 provides a minimum of diversity representative of genomic heterogeneity (if any is present). In addition, this lower limit is useful -in the sample under test- for the detection of a potential contamination from another cell line with higher sensitivity, as a low-level of contamination -e.g. 10%- may fail altogether to be represented in a cell pool with a lower number of cells, or anyway result in a sample where the minor contaminant is under-represented with respect to the real % in the population, thus potentially reducing the overall sensitivity in detecting said contamination. The higher limit of 1500 (i.e. equivalent to lOng) is preferable to ensure good WGA amplification without inhibition which might occur with overloading of the WGA reaction with input DNA or inhibitory effect of an entire cell lysate when starting directly from cells without DNA purification.

Example 17 - Application to allogenic hematopoietic cell transplantation.

In another preferred embodiment, the method according to the present disclosure is used for the assessment of endothelial cell origin in patients of allogenic hematopoietic cell transplantation (allo-HSCT).

In a preferred embodiment of the present disclosure, isolation of individual endothelial cells is carried out from either

1.FFPE sections, following disaggregation, staining with endothelial cell markers, such as CD146, and single-cell sorting, such as for example with DEPArray™.

2.peripheral blood, following enrichment and staining of Circulating Endothelial Cells (CEC) using CELLSEARCH® AutoPrep and CEC kit, and single-cell sorting, such as for example with DEPArray™.

A first reference sample is provided, comprising germline DNA from the host. Single endothelial cells are isolated from the patients and their similarity score with the reference host sample is assessed. If the cell under test is classified as self it means that it is confirmed of host origin, whereas if classified as unrelated is classified as belonging to the unrelated donor.

The method can be applied using also a kinship analysis to identify the donor cells in case the donor is linked to the host by a kinship relationship.

If, in addition, a donor germline DNA sample is available, a second reference sample can be generated as confirmation of the classification.

**Additional general details and considerations which apply across different applications**

Locus to fragment-length univocal relationship in DRS-WGA

More in detail, the method according to the present disclosure exploits the fact that in DRS-WGA, such as the Ampli1™ WGA, each locus in the genome is represented in the WGA library only in fragments having a specific length in base-pairs. This property may be designated "Locus to Fragment-Length Univocal Relationship" (L2FLUR). Considering a general normal locus, e.g. a locus for a polymorphic SNP, said locus will be represented only in a fragment of a given length, equal to the size of the corresponding fragment (measured on either of the single-strands) following digestion by the restriction enzyme, plus double the length of the universal WGA adaptors (the length of the LIB1 primer in case of Ampli1 WGA). When the WGA is sequenced following library preparation according to Ampli1 LowPass kits, a predictable additional length is introduced linked to the sequencing adaptors and barcodes lengths, which are known.

Reproducibility and Reduced representation of the genome

In the method according to the present disclosure, the property of DRS-WGA combined with the random fragmentation-free library preparation is exploited to produce a reduced representation of the genome (with respect to the original size of the samples reference genome), whereby the low-pass sequencing data, for a given number of reads, increases the probability to cover the same fragments across different samples, with respect to when a random process is inherent in the WGA (e.g. as with WGA methods using Multiple Displacement Amplification or DOP-PCR) and/or in the sequencing library preparation (e.g. by random fragmentation or tagmentation).

In other words, a deterministic subsampling of the reference genome occurs. The term "deterministic" is essential, in that - for any given number of reads - the overlap in genomic loci covered across any two paired samples is higher, thus increasing the number of highly polymorphic loci available for measuring the similarity of the DNA of those samples.

It is worth noting that the approach is flexible in that different deterministic enzymes may be suitable depending on the desired resolution and/or sequencing platform and sequencing protocol used. For example, different frequent cutters may be used. In the examples of Ampli1 WGA, the TTAA motif is the Restriction Site. Other four-base cutters may be used to cut at different Restriction Site, such as GTAC, CTAG, obtaining a different distribution of fragments, allowing one to tune the number of loci in common across different samples for a given number of reads.

When the DRS-WGA is first purified after the primary PCR, a first size-selection occurs, whereby shorter fragments of the WGA are removed along with free primers. Advantageously, the method uses a further step of selection. This additional step of selection can be achieved by either size-selecting certain fragments from the primary WGA and/or generating the massively parallel sequencing library by a method which restricts the sequenceable fragments. For example, Ampli1 LowPass kits include an inherent size selection step which is sufficient to positively impact the process. In WO2017/178655, a size selection on a gel is carried out. In WO2019/016401, successive steps of purification using SPRI-beads effectively produce a first size selection, whereby the length of base-pairs is restricted to a range substantially depending on the SPRI-beads concentration. In addition, the sequencer may also introduce a size selection per se, as longer fragments will generate sequence data with lower and lower efficiency (e.g. due to emulsion PCR efficiency in Ion Torrent, or bridge PCR for cluster formation in Illumina platforms).

In DRS-WGA there is also a deterministic relationship between the average size of the sequencing library and the subsampling ratio of the reference genome.

An *in-silico* analysis, carried out on the TTAA digest of the human reference genome hg19, yields a total of about 19M fragments including all chromosome sequences, which would translate to 38M fragments on a normal diploid human genome. By way of example, selecting *in-silico,* fragments in the range 175-225bp will be only 1,252,559, covering approximately a total of 248M bases out of 3.09B bases, i.e. 8.02% of the human reference genome. See Table 1 below, in which number of fragments, total base-pairs and reduction ratio (%) are listed for different ranges of selection by size. This subsampling can be designated the Reduction Ratio (RR) .

Table 1

Reduction ratio depending of fragments size selection

| Range | N. Fragments | Tot. bps | Reduction Ratio |
|---|---|---|---|
| 75-125 | 3,057,163 | 298,483,600 | 9.64 |
| 175-225 | 1,252,559 | 248,367,191 | 8.02 |
| 275-325 | 703,011 | 210,389,610 | 6.80 |
| 375-425 | 390,419 | 155,603,924 | 5.03 |
| 475-525 | 217,861 | 108,653,407 | 3.51 |
| 725-775 | 68,581 | 51,428,399 | 1.66 |
| 975-1025 | 24,091 | 24,070,638 | 0.78 |

In a preferred embodiment of the present disclosure, the objective is to obtain a good resolution in the pair-wise similarity score across samples. To increase the resolution for a given number of reads which may be available for each sample (linked to the cost of sequencing per sample), the overlap in covered base pairs between any two samples is relevant, as only regions covered in both samples are compared. Thus, increasing the base-pairs range of fragments sequenced may help reduce the diversity of fragments, increasing the overlap between different samples.

There are however trade-offs depending on the application. In certain embodiments of the present disclosure, besides the identification of the DNA origin of a sample, the low-pass whole genome sequencing data serves also a dual purpose of generating a genome-wide copy number profile of the samples itself, as it is the case for NIPD application or for the cell-free spent culture medium of embryos.

In this case, a fragment range of the similar width but centered on shorter fragments increase diversity and can produce better results and resolution for the copy-number caller, as there are higher number of fragments contributing to the read counts in a given genomic window.

Size selection of fragments

Different size-selection techniques may also be used to achieve the desired Reduction Ratio, depending on the elected number of sequencing reads per sample and/or resolution. For a given average fragment length - smaller or larger number of total fragments can be obtained selecting a respectively smaller or larger band centered on that average fragment length.

Instruments like the Pipping prep (Sage Science) may be used to have a tighter control on the fragment length distribution and, using an analogy to passband filters, also in having higher Q factor defined as

$$Q = Fcenter/DeltaF = [(Fmin+FMAX)/2]/(FMAX-Fmin)$$

where

$$Fcenter = (Fmin+FMAX)/2 \text{ is the average size of Fragments}$$

DeltaF = FMAX-Fmin is the width of the range of fragment sizes

Fmin is the size of fragments below which fragments are represented at a conventional relative level (e.g. 1/10=10%) or less with respect to the normalized, in-band, peak number of fragments per bin.

FMAX is the size of fragments above which fragments are represented at the same conventional relative level or less with respect to the normalized in-band peak number of fragments per bin.

With Illumina sequencing, the sequencing mode is preferably paired-end sequencing, as the covered genome increases and thus the number of loci per-million read-pairs increases, augmenting the resolution. However, when the size selected for sequencing gets below a certain size, the paired-end sequencing will not increase the coverage as the two paired reads overlap completely.

With Ion Torrent sequencing, higher read lengths will proportionally increase the covered genome and thus the number of loci per-million reads increases, augmenting the resolution. In the Ampli1 LowPass IonTorrent kit (Menarini Silicon Biosystems), the barcoded pooled samples are size selected, on a gel or with other methods like Pippin Prep. The choice of different Q factor and average fragment length can provide different resolutions on a per million reads basis.

One advantage of pooling the samples and size-selecting the library for sequencing thereafter is that all samples will have the same distribution of fragment lengths, and in turn this will maximize the overlap of covered genome across different samples, as required to provide for a higher number of highly polymorphic loci for the comparison.

On the other hand, when using the Ampli1 LowPass kit for Illumina, the different LowPass libraries are at first size-selected and then pooled obtaining slightly different size-selections across different samples, thus reducing the covered genome across different samples.

A size-selection after library pooling, although not mandated by the standard protocol, may be employed to increase the overlap across samples, which may be beneficial in analysis based on controls.

It is however important that there is overlap between the distribution of DRS-WGA fragments sequenced across different samples, as reduction of overlap in fragment distribution may decrease the number of polymorphic loci in common for pair-wise similarity score assessment, in turn reducing the resolution of the method.

According to the present disclosure, the combination of DRS-WGA and LPWGS leads to a reduced representation from the input samples. By sequencing with NGS, this reduced representation libraries of the reference genome, in turn shrinks the covered genome in the selected (or any way sequenceable) base-pair range, and an effectively higher overlap of the covered genome across different samples, on a per reads basis, is obtained

This effect can be exploited according to the present disclosure in different ways, depending on the situation.

Preferably, the library preparation from the DRS-WGA is one of the methods disclosed in WO2017/178655 or WO2019/016401.

Similarity-score thresholding and identity calling

Optionally, the similarity-score obtained from previous steps may be thresholded to define sample classes. In most cases, the number of polymorphic loci available for comparison across two samples will increase at higher read depths. To allow the thresholding of the similarity score using a precomputed value, the number of mapped reads in each sample is preferably normalized to a fixed number of reads. Such normalization is performed by randomly sampling reads, mapping to the reference genome, until the desired number is reached (preferably contained in the range going from 100,000 mapped reads to 10,000,000 mapped reads).

In a preferred embodiment of the present disclosure, a "self" relationship between two samples is called if the similarity-score is higher than a first selected threshold.

In a preferred embodiment of the present disclosure, an "unrelated" relationship between two samples is called if the similarity-score is lower than a second selected threshold.

In the application to non-invasive prenatal diagnosis, a "kin" relationship between two samples, is called if the similarity-score is comprised between a third threshold, equal or lower to said first threshold, and a fourth threshold, equal or higher than said second threshold.

In the application to forensic human identification, a "mixed" relationship between two samples, is called if the similarity-score is comprised between a third threshold, equal or lower to said first threshold, and a fourth threshold, equal or higher than said second threshold.

## Claims

1. A method for analyzing the degree of similarity of at least two samples in a plurality of samples comprising genomic DNA, the method comprising the steps of:

   a) providing a plurality of samples comprising genomic DNA;
   b) carrying out, separately on each sample, a deterministic restriction-site whole genome amplification (DRS-WGA) of said genomic DNA;
   c) preparing a massively parallel sequencing library using a fragmentation-free, sequencing-adaptor/WGA fusion-primer PCR reaction from each product of said DRS-WGA;
   d) carrying out low-pass whole genome sequencing at a mean coverage depth of < 1x on said massively parallel sequencing library;
   e) aligning for each sample the reads obtained in step d) on a reference genome;
   f) extracting for each sample the allelic content at a plurality of polymorphic loci;
   g) calculating a pair-wise similarity score for the at least two samples as a function of the allelic content measured at said plurality of loci;
   h) determining the degree of similarity of the at least two samples on the basis of the similarity score.

2. The method according to claim 1, wherein said low-pass whole genome sequencing is carried out at a coverage < 0.01x, preferably at a coverage < 0.05x, more preferably at a coverage < 0.1x, even more preferably at a coverage < 0.5x.

3. The method according to claim 1 or 2, wherein said plurality of polymorphic loci comprises polymorphic loci with average heterozygosity > 0.499, preferably with average heterozygosity > 0.49, more preferably with average heterozygosity > 0.4, even more preferably with average heterozygosity > 0.3, the most preferably with average heterozygosity > 0.2.

4. The method according to any one of claims 1-3, wherein said plurality of polymorphic loci comprises > 200,000 loci, preferably > 300,000 loci, more preferably > 500,000 loci, even more preferably > 1,000,000 loci.

5. The method according to any of claims 1-4, wherein said pair-wise similarity score is calculated by computing the correlation of the B-allele frequency across loci covered by at least one read in the at least two samples.

6. The method according to any of claims 1-4, wherein said pair-wise similarity score is calculated by computing the mean concordance value across loci covered by at least one read in both paired samples, wherein the concordance value for each locus is assigned one the following values:

   A1) 1 if the alleles called are identical; and
   B1) 0 if the alleles called are different; or
   A2) 1 if the alleles called are identical;
   B2) 0 if the alleles called are completely different; and
   C2) 0.5 if the alleles called are partially overlapping.

7. The method according to any of the preceding claims, further comprising defining a group of clusters of samples sharing a common property selected from the group consisting of the identity of the one individual (or more individuals) substantially contributing with DNA to the samples of a cluster, or the property of containing insufficient quantities of DNA and/or the property of containing highly degraded DNA or DNA of uncertain origin.

8. The method according to claim 7, wherein the at least two samples are assigned to at least one cluster by means of an algorithm using as input said pair-wise similarity score.

9. The method according to claim 8, wherein the algorithm is a hierarchical clustering algorithm.

10. The method according to claim 8, wherein the number of said clusters is calculated by

    a) selecting a number of first-iteration clusters maximizing the average silhouette score;
    b) for each one of said first-iteration clusters, computing the silhouette score of each of said samples belonging to the first-iteration cluster, wherein samples belonging to the cluster having a silhouette score lower than a fixed threshold comprised in the range 0.19-0.21, are assigned to a new cluster.

11. The method according to claim 10, wherein said group of clusters comprises one or more identity-clusters comprising samples containing DNA from only one and the same individual.

12. The method according to claim 11, wherein, in the presence of more identity clusters, the cardinality of said plurality of identity-clusters corresponds to the number of individual DNA contributors in said plurality of samples.

13. The method according to any of claims from 8 to 12, further comprising defining a group of mixed-identity-clusters, each of said mixed-identity clusters comprising samples containing DNA from at least two individuals.

14. The method according to claim 13, further comprising defining at least one no-call-cluster, comprising samples containing DNA from uncertain origin.

15. The method according to any of claims from 8 to 14, wherein said plurality of samples comprises at least one reference sample and said group of identity clusters includes at least one reference-cluster, comprising said reference sample.

16. The method according to claim 15, wherein said at least one reference sample is a sample from a pregnant female-parent individual.

17. The method according to claim 16, wherein said group of identity-clusters further contains at least one kin-cluster composed by samples from at least one fetus from the ongoing pregnancy of said female-parent individual.

18. The method according to claim 17, wherein said kin-cluster is partitioned in a plurality of fetal-clusters composed of samples which contain DNA from only one and the same fetus.

19. The method according to claim 1 or 15, wherein said plurality of samples comprises at least a reference sample containing DNA from a female-parent individual, and at least one other embryonic sample from said plurality of samples is selected from the group consisting of:

    a) samples containing DNA from an embryo derived from said female-parent individual; and
    b) samples containing DNA from a spent embryo-culture medium obtained from an embryo of said female-parent individual.

20. The method according to claim 19, further comprising carrying out a pre-implantation genetic screening on said embryo by analyzing genome-wide chromosomal aberrations from said low-pass whole genome sequencing data from said at least one other embryonic sample using a contamination factor corresponding to maternal contamination measured on said at least one other embryonic sample as a function of said pair-wise similarity of said at least one other embryonic sample from said female-parent individual sample.

21. The method according to claim 15, wherein said plurality of samples comprises at least a reference sample containing DNA from a female-parent individual, and at least one other sample containing DNA from a cell-free DNA sample.

22. The method according to claim 21, further comprising carrying out a non-invasive prenatal testing on said cell-free DNA sample by analyzing genome-wide chromosomal aberrations from said low-pass whole genome sequencing data from said at least one cell-free DNA sample using a correction factor corresponding to the fetal fraction measured on said at least one cell-free DNA sample as a function of said pair-wise similarity with female-parent reference sample.

23. The method according to claim 15, wherein said plurality of samples comprises at least a reference sample containing DNA from a female-parent individual, and at least one other prenatal sample containing DNA from chorionic villi, amniotic fluid or products of conception.

24. The method according to claim 23, further comprising carrying out a prenatal testing on said prenatal samples by analyzing genome-wide chromosomal aberrations from said low-pass whole genome sequencing data from said at least one prenatal sample using a correction factor corresponding to the maternal or exogenous contamination measured on said at least one prenatal sample as a function of said pair-wise similarity with female-parent reference sample.

25. The method according to claim 15, in particular for cell line authentication, wherein a plurality of reference clusters is generated from a plurality of samples of DNA from cell lines, and said group of identity clusters further contains at least one samples from a cell line to be authenticated.

26. The method according to claim 17, in particular for non-invasive paternity testing, wherein said at least one reference sample comprises a male-parent reference sample containing DNA only from said male-parent, and said at least one reference-cluster further comprises a male-parent identity cluster including said male-parent sample, wherein:

(i) if the kin-sample similarity score with respect to the male-parent sample is consistent with kinship the paternity is confirmed
(ii) if kin-sample similarity score with respect to the male-parent sample is consistent with an unrelated individual the paternity is not confirmed.

27. The method according to claim 17, in particular for non-invasive molar pregnancy assessment, wherein said at least one sample comprises at least one circulating trophoblastic cell sample and wherein, if said trophoblastic cell sample similarity score with respect to the female-parent samples is consistent with unrelated samples, a complete mole is confirmed.

28. The method according to claim 27, wherein said at least one sample comprises a plurality of trophoblastic cell samples and wherein:

(i) if the similarity score among said trophoblastic cell samples exceeds the expected 99th percentile of the expected similarity score for self samples a P1P1 homozygous paternal mole is confirmed.
(ii) if the similarity score among said trophoblastic cell samples is consistent with the expected similarity score for self samples a P1P2 heterozygous paternal mole is confirmed.

29. The method according to claim 25, wherein said at least one sample further comprises a male-parent sample and the similarity score among said trophoblastic cell samples is consistent with the expected similarity score for self samples, wherein:

(i) if said trophoblastic cells samples similarity score with respect to the male-parent sample is consistent with the expected similarity score for self samples, a P1P2 heterozygous paternal mole is confirmed.
(ii) if said trophoblastic cells samples similarity score with respect to the male-parent sample is lower than the 1st percentile of the expected similarity score for self samples, a P1P2 heterozygous paternal mole is not confirmed.

30. The method according to any one of claims 1-6, further comprising classifying samples selected from a plurality of samples, based on predefined classes using a machine-learning classifier using as input said pair-wise similarity score.

31. The method according to claim 30, wherein the machine-learning classifier is a random forest classifier.

32. The method according to claim 30 or 31, wherein the machine-learning classifier uses as further input at least one value, measured on said low-pass whole-genome sequencing data, selected from the group comprising:

a) DLRS: derivative log ratio spread;
b) R50: percentage of WGA fragments covered by 50% of sequenced reads over total WGA fragments covered by at least one read;
c) YFRAC: fraction of reads mapping to chromosome Y;
d) Aberrant: percentage of genome corresponding to gains or losses respect to median cell ploidy;
e) Chr13: ploidy of chromosome 13;
f) Chr18: ploidy of chromosome 18;
g) Chr21: ploidy of chromosome 21;
h) RSUM: mean absolute deviation from nearest integer copy number level, calculated on the copy number aberration event with highest absolute deviation from median cell ploidy;
i) Mix_score: RSUM z-score, calculated on the copy number aberration event with highest absolute deviation from median cell ploidy; and
f) Deg_score: number of small loss events (< 10 Mbp, which is common in degraded samples).

33. The method according to any of claims from 30 to 32, wherein at least one of the samples is a reference sample.

34. The method according to claim 33, wherein said at least one reference sample comprises a sample from a pregnant female-parent individual.

35. The method according to claim 34, wherein said plurality of samples comprises at least one sample classified as "kin" with respect to the female-parent reference, representing the sample from a fetus from an ongoing pregnancy of said female-parent individual.

36. The method according to any one of claims 30 to 33, wherein said plurality of samples comprises at least a reference sample containing DNA from a female-parent individual, and at least one other embryonic sample, classified as "non-self" with respect to the female-parent reference, from said plurality of samples is selected from the group consisting of:

a) samples containing DNA from an embryo derived from said female-parent individual; and
b) samples containing DNA from a spent embryo-culture medium obtained from an embryo of said female-parent individual.

37. The method according to claim 36, further comprising carrying out a pre-implantation genetic screening on said embryo by analyzing genome-wide chromosomal aberrations from said low-pass whole genome sequencing data from said at least one other embryonic sample using a contamination factor corresponding to maternal contamination measured on said at least one other embryonic sample as a function of said pairwise similarity of said at least one other embryonic sample from said female-parent individual sample.

38. The method according to claim 35, in particular for non-invasive paternity testing, wherein said at least one reference sample further comprises a male-parent reference sample containing DNA only from said male-parent, and said plurality of samples further comprises samples, wherein:

(i) paternity is confirmed if they are classified as "self" with respect to the male-parent reference sample
(ii) paternity is not confirmed if they are classified as "unrelated" with respect to the male-parent reference sample.

39. The method according to claim 34, in particular for non-invasive molar pregnancy assessment, wherein said at least one sample comprises at least one circulating trophoblastic cell sample and wherein, if said trophoblastic cell sample is classified as "unrelated" with respect to the female-parent reference, a complete hydatiform mole of paternal origin is confirmed.

40. The method according to claim 39, wherein said at least one sample comprises a plurality of trophoblastic cell samples, which are classified as "self" with respect to each other, and wherein:

(i) if their similarity score exceeds the expected 99th percentile of the expected similarity score for "self" samples, a P1P1 homozygous hydatiform mole of paternal origin is confirmed.
(ii) if their similarity score is consistent with the expected similarity score for "self" samples, a P1P2 heterozygous hydatiform mole of paternal origin is confirmed.

41. The method according to claim 40, wherein said at least one sample further comprises a male-parent sample, wherein said male-parent sample is classified as "self" with respect to at least one sample of said plurality of trophoblastic cell samples, and wherein:

(i) if said trophoblastic cells samples similarity score with respect to the male-parent sample is consistent with the expected similarity score for "self" samples, a P1P2 heterozygous hydatiform mole of paternal origin is confirmed.
(ii) if said trophoblastic cells samples similarity score with respect to the male-parent sample is lower than the 1st percentile of the expected similarity score for "self" samples, a P1P2 heterozygous hydatiform mole of paternal origin is not confirmed.

FIG. 1

FIG. 2

FIG. 3A

FIG. 3B

AvHet threholds:
500,000 reads: 0.46
5M reads: 0.49

# FIG. 4B

# FIG. 4A

# FIG. 4E

# FIG. 4D

## FIG. 4C

## FIG. 4F

FIG. 5A
kin-self

FIG. 5B
kin-unrelated

FIG. 5C
kin-self

FIG. 5D
kin-unrelated

thresholds at different AvHet for which PPV > 0.999.

FIG. 6

FIG. 7

FIG. 8

# FIG. 9A

# FIG. 9B

# FIG. 9C

FIG. 10A

FIG. 10B

FIG. 10C

FIG. 11A

FIG. 11B

FIG. 12

EP 4 521 411 A2

FIG. 13A

FIG. 13B

FIG. 13C

FIG. 14

FIG. 15

## REFERENCES CITED IN THE DESCRIPTION

**Patent documents cited in the description**

- EP 2152859 B1, Manaresi
- WO 2000017390 A
- WO 2017178655 A
- WO 2019016401 A1
- WO 2021019459 A1
- WO 2019016401 A

**Non-patent literature cited in the description**

- **FONTANA**. Isolation and genetic analysis of pure cells from forensic biological mixtures: The precision of a digital approach. *Forensic Sciences International: Genetics*, 2007, http://dx.doi.org/10.1016/j.fsigen.2017.04.023
- **K. ANSLINGER** ; **B. BAYER**. Whose blood is it? Application of DEPArray™ technology for the identification of individual/s who contributed blood to a mixed stain. *Int J Legal Med.*, March 2019, vol. 133 (2), 419-426
- **K. HUFFMAN** ; **E. HANSON** ; **J BALLANTYNE**. Recovery of single source DNA profiles from mixtures by direct single cell subsampling and simplified micromanipulation. *Science & Justice*, January 2021, vol. 61 (1), 13-25
- **VOSSAERT L** ; **WANG Q** ; **SALMAN R et al.** Validation Studies for Single Circulating Trophoblast Genetic Testing as a Form of Noninvasive Prenatal Diagnosis. *American Journal of Human Genetics*, 2019, vol. 105 (6), 1262-1273
- **L.D. JEPPESEN et al.** Cell-based non-invasive prenatal diagnosis in a pregnancy at risk of cystic fibrosis. *Prenatal Diagnosis*, 2020, 1-7
- **ZHUO X** ; **WANG Q** ; **VOSSAERT L** ; **SALMAN R** ; **KIM A** ; **VAN DEN VEYVER I et al.** Use of amplicon-based sequencing for testing fetal identity and monogenic traits with Single Circulating Trophoblast (SCT) as one form of cell-based NIPT. *PLoS ONE*, 2021, vol. 16 (4), 0249695, https://doi.org/10.1371/-journal.pone.0249695)
- **SUNDE L et al.** Hydatidiform mole diagnostics using circulating gestational trophoblasts isolated from maternal blood. *Mol Genet Genomic Med.*, 2020, 1565, https: / /doi. org/10 .1002/mgg3 .1565
- **PENACK O. et al.** The importance of neovascularization and its inhibition for allogeneic hematopoietic stem cell transplantation. *Blood*, 21 April 2011, vol. 117 (16), 4181-4189
- **PENG XL** ; **JIANG P.** Bioinformatics Approaches for Fetal DNA Fraction Estimation in Noninvasive Prenatal Testing.. *Int J Mol Sci.*, 20 February 2017, vol. 18 (2), 453
- **SEJOON LEE et al.** NGSCheckMate: software for validating sample identity in next-generation sequencing studies within and across data types. *Nucleic Acids Research*, 2017, vol. 45 (11)
- **HODGKINSON C.L. et al.** *Nature Medicine*, 2014, vol. 20, 897-903
- **FERRARINI et al.** *PLoSONE*, vol. 13 (3), 0193689
- **STOECKLEIN et al.** *Am J Pathol.*, July 2002, vol. 161 (1), 43-51
- **ARNESON et al.** *ISRN Oncol.*, 14 March 2012, vol. 2012, 710692
- **MANGANO, C.** ; **FERRARINI, A.** ; **FORCATO, C. et al.** Precise detection of genomic imbalances at single-cell resolution reveals intra-patient heterogeneity in Hodgkin's lymphoma. *Blood Cancer J*, 2019, vol. 9, 92, https://doi.org/10.1038/s41408-019-0256-y
- **BOEVA, V. et al.** *Bioinformatics*, 01 February 2012, vol. 28 (3), 423-5
- **MANGANO C. et al.** Precise detection of genomic imbalances at single-cell resolution reveals intra-patient heterogeneity in Hodgkin's lymphoma. *Blood Cancer Journal*, 2019, vol. 9 (92)